# FASCICULE DE BREVET EUROPEEN

(11) **EP 2 473 629 B1**
(45) Date de publication et mention de la délivrance du brevet: **25.02.2015**
(21) Numéro de dépôt: 10763790.2
(22) Date de dépôt: 01.09.2010
(51) Int. Cl.: C12Q 1/68

(54) **IDENTIFICATION DU TROPISME CELLULAIRE DE VIRUS**
IDENTIFIZIERUNG VON VIRUS TROPISMUS
IDENTIFICATION OF THE VIRAL CELLULAR TROPISM

(30) Priorité: 04.09.2009 FR 0956045
(43) Date de publication de la demande: 11.07.2012
(73) Titulaire: Centre National de la Recherche Scientifique (CNRS), 75016 Paris (FR); Assistance Publique Hôpitaux De Paris, 75184 Paris Cedex 04 (FR); Université Paris Diderot - Paris 7, 75205 Paris Cedex 13 (FR); Université Montpellier 2, Sciences et Techniques, 34095 Montpellier Cedex 5 (FR)
(72) Inventeur: LECELLIER, Charles-Henri, F-34920 Le Cres (FR); COURGNAUD, Valérie, F-34090 Montpellier (FR); BOUTTIER, Manuella, F-34070 Montpellier (FR); DESCAMPS, Diane, F-75015 Paris (FR); COLLIN, Gilles, F-78300 Poissy (FR)
(74) Mandataire: Jacobson, Claude
(86) Numéro de dépôt international: PCT/FR2010/051817
(87) Numéro de publication internationale: WO 2011/027075

(56) Documents cités:
- WO-A1-2009/033185
- VAN BAELEN ET AL: "HIV-1 coreceptor usage determination in clinical isolates using clonal and population-based genotypic and phenotypic assays", JOURNAL OF VIROLOGICAL METHODS, ELSEVIER BV, NL LNKD- DOI:10.1016/J.JVIROMET.2007.06.003, vol. 146, no. 1-2, 3 novembre 2007 (2007-11-03), pages 61-73, XP022327138, ISSN: 0166-0934
- SOULIÉ C ET AL: "[HIV tropism assays when first CCR5-antagonist becomes available]", MÉDECINE ET MALADIES INFECTIEUSES MAR 2008 LNKD- PUBMED:18455056, vol. 38 Suppl 1, mars 2008 (2008-03), pages S7-11, XP002578279, ISSN: 1769-6690

## Description

### Domaine de l'invention

La présente invention comme définie par les revendications 1-16 concerne une méthode de caractérisation du tropisme cellulaire d'un virus, notamment du Virus d'Immunodéficience Humaine (VIH), et en particulier de la capacité du virus VIH à utiliser les récepteurs CXCR4 et/ou CCR5 pour entrer dans les cellules.

### Arrière-plan technique

L'entrée du virus VIH dans les cellules implique plusieurs protéines virales dont les protéines d'enveloppe gp41 et gp120. La première étape du cycle de réplication du virus VIH fait intervenir la fixation du virus aux lymphocytes T4 auxiliaires par interaction de la protéine gp120 avec la protéine cellulaire CD4. En outre, pour que la fusion des membranes virale et cellulaire se produise, le virus VIH doit interagir avec un co-récepteur cellulaire. Les co-récepteurs les plus importants *in vivo* sont les récepteurs de chimiokines CXCR4 et CCR5. L'utilisation des différents co-récepteurs est associée à l'évolution du déficit immunitaire et, par conséquent, de l'infection : au début de l'infection, les virus dits R5 interagissent avec le co-récepteur CCR5, puis à un stade plus avancé, certains virus utilisent le co-récepteur CXCR4 (virus dits X4). A ce stade, la population virale comprend soit un mélange de virus R5 et X4, soit des virus à double tropisme R5/X4. Dans certains cas, les virus R5 peuvent induire directement l'apparition d'un SIDA, toutefois c'est l'apparition de virus X4 qui est généralement associée au développement de la maladie. Il est donc essentiel de pouvoir détecter précocement l'apparition des virus X4 chez le patient.

Ainsi, un certain nombre de méthodes ayant pour but de déterminer le tropisme cellulaire des virus VIH ont été développées.

Par exemple, le test TROFILE (MONOGRAM) est un test phénotypique du virus VIH proposé par la société Monogram Biosciences et Pfizer. Tout d'abord, une librairie de vecteurs contenant les régions codant l'enveloppe des virus VIH d'un patient est réalisée. Ces vecteurs sont ensuite amplifiés et les régions codant l'enveloppe sont clonées dans un vecteur exprimant un virus VIH dépourvu de protéine d'enveloppe et exprimant le gène de la luciférase. Enfin, les virus recombinants obtenus à partir de ces vecteurs sont utilisés pour infecter des cellules exprimant CD4 et CCR5 ou CXCR4. La capacité d'un virus à infecter ces cellules est déterminée par mesure de l'émission de lumière produite par la luciférase. Ce test présente plusieurs inconvénients, comme signalé en novembre 2007 par le TRT-5 à l'Afssaps et à la HAS (Haute Autorité de la Santé). Il a tout d'abord un coût très élevé. De plus, le délai de réception des résultats est de quatre à cinq semaines, ce qui n'est pas compatible avec une prise de décision rapide en cas de changement de traitement. En outre, il n'est pas impossible qu'un changement de tropisme viral puisse intervenir chez certains patients dans un tel délai. Par ailleurs, aucun test Trofile n'est disponible pour les virus de types VIH-2.

Il existe donc un réel besoin de tests alternatifs simples, rapides, fiables et peu coûteux permettant de déterminer le tropisme cellulaire des virus VIH. L'objet de la présente invention est de fournir de tels tests.

### Résumé de l'invention

La présente invention comme définie par les revendications 1-16 découle de la mise en évidence inattendue, par les inventeurs, que l'expression de miRNA dans une cellule susceptible d'être infectée par un virus VIH est modulée en fonction du co-récepteur utilisé par le virus VIH pour entrer dans la cellule.

Ainsi, la présente invention concerne une méthode *in vitro* d'identification de microARN, ou de leurs ARNm cibles, dont l'expression, lors de l'infection de cellules par un virus utilisant un récepteur cellulaire et au moins un co-récepteur cellulaire pour entrer dans la cellule, est spécifiquement modifiée en fonction du co-récepteur cellulaire utilisé par le virus pour son entrée dans les cellules, comprenant :
i) déterminer les niveaux d'expression de microARN dans une cellule test, exprimant un récepteur, un premier co-récepteur et au moins un autre co-récepteur, après infection respectivement par un premier virus utilisant le premier co-récepteur et par au moins un autre virus utilisant un autre co-récepteur ;
ii) identifier les microARN dont le niveau d'expression est modulé lors de l'infection par chacun des virus par rapport au niveau d'expression dans des cellules non infectées ;
iii) comparer les microARN ainsi identifiés ;
iv) sélectionner les microARN dont la modification du niveau d'expression est spécifique de l'utilisation d'un co-récepteur ;
v) éventuellement identifier les ARNm cibles des micro-ARN ainsi sélectionnés.

L'invention concerne également méthode *in vitro* d'identification d'un co-récepteur cellulaire utilisé par un virus utilisant un récepteur cellulaire et au moins un co-récepteur cellulaire pour entrer dans une cellule, chez un patient infecté par le virus, comprenant :
i) mettre en contact un échantillon du patient susceptible de contenir le virus avec une cellule test exprimant un récepteur cellulaire du virus et au moins un co-récepteur cellulaire du virus ;
ii) déterminer le niveau d'expression d'au moins un miRNA et/ou d'au moins un ARNm cible d'un miRNA dans la cellule test ;
iii) comparer le niveau d'expression à une valeur prédéterminée ;
iv) en déduire si le virus utilise ou non un co-récepteur cellulaire exprimé par la cellule test.

### Description de l'invention

Le terme « miRNA » ou « microARN » se réfère à une classe d'ARN, généralement de 20 à 25 nucléotides de long, impliqués dans la régulation post-transcriptionelle de certains gènes spécifiques en dégradant ou bloquant la traduction de l'ARNm issu de la transcription de ces gènes. Par « ARNm cible » d'un miRNA, on entend un ARNm dont on sait ou dont on a déterminé qu'il est dégradé ou dont la traduction est bloquée par ledit miRNA. Les miRNAS sont notamment décrits dans Griffiths-Jones ((2004) Nucleic Acids Res 32:D109-D111), dans Griffiths-Jones et al. ((2008) Nucleic Acids Res 36:D154-D158)et dans la base de donnée sur les miRNA (miRBase, http://microARN.sanger.ac.uk).

L'expression « virus » telle qu'utilisée ici comprend tous les types de virus. En particulier le virus peut être sélectionné dans le groupe des virus dont des variants ou des espèces sont plus ou moins pathogènes, par exemple les rétrovirus en particulier le virus VIH, les virus de la grippe, les coronavirus, les virus de la rougeole, les herpesvirus (dont les virus EBV, Simplex et CMV), les papillomavirus. Préférentiellement, le virus est un rétrovirus sélectionné parmi les retrovirus humains notamment VIH, HTLV-I et XMRV. Encore plus préférentiellement, le virus est le VIH et en particulier les virus VIH-1 et VIH-2 (notamment décrits dans la base de donnée *HIV databases,* http://www.hiv.|an|.gov/content/index). Si le virus selon l'invention est le VIH, les virus utilisés pour réaliser les infections peuvent, par exemple, être des virus prototypes comme les virus VIH-1, NL4.3, VIH-2 ROD ou VIH-1 NLAD8 ou des virus d'un patient. Les virus VIH selon l'invention peuvent utiliser un ou plusieurs co-récepteurs pour entrer dans les cellules cibles. Préférentiellement, dans les méthodes d'identification de micro-ARN selon l'invention, les virus VIH utilisés n'utilisent qu'un seul type de co-récepteur pour entrer dans une cellule.

Le terme « patient » désigne un être humain infecté par un virus. Préférentiellement, le virus est le VIH. Le patient peut alors avoir éventuellement développé un SIDA (Syndrome d'Immuno-Déficience Acquise). Eventuellement, le patient est sous traitement anti-rétroviral, par exemple sous traitement HAART (traitement anti-retroviral hautement actif (« Highly Active Anti-Retroviral Therapy »)).

Les termes « récepteur » et « récepteur cellulaire » selon l'invention désigne une structure de surface cellulaire, en générale une protéine, intervenant dans la reconnaissance d'une cellule cible par un virus et aboutissant en générale à la fixation de ce virus à la cellule cible. Les termes « co-récepteur » et « co-récepteur cellulaire » regroupe l'ensemble des protéines cellulaires de surface participant à l'entrée du virus à l'exclusion du récepteur cellulaire.

Lorsque le virus est le VIH, les termes « co-récepteur » et « co-récepteur cellulaire » regroupent plus spécifiquement l'ensemble des protéines cellulaires de surface participant à l'entrée du virus en plus de l'interaction entre le virus et le récepteur cellulaire CD4. L'entrée d'un virus VIH dans une cellule hôte implique la fusion entre les membranes cellulaire et virale. En particulier, le co-récepteur peut être sélectionné dans le groupe constitué de CXCR4, CCR5, CCR3, CCR2, CCR1, CCR4, CCR8, CCR9, CXCR2, STRL33, V28, gpr1, gpr15 et ChemR23. Préférentiellement, le co-récepteur est CCR5 ou CXCR4.

CXCR4 est également connu sous les noms de Fusin, LESTR et NPY3R. Le gène CRCR4 désigne ici préférentiellement la séquence du gène CXCR4 humain dont la séquence d'ARNm peut, par exemple, être la SEQ ID NO : 1 ou tout variant allélique ou polymorphique de celle-ci ainsi que les séquences orthologues présentes dans d'autres espèces. Le gène CXCR4 code la protéine CXCR4 pouvant avoir la séquence représentée par SEQ ID NO : 2 ou n'importe quel variant naturel de celle-ci.

CCR5 est également connu sous les noms de CKR-5 et CMKRB5. Le gène CCR5 désigne ici préférentiellement la séquence du gène CCR5 humain dont la séquence d'ARNm peut, par exemple, être la SEQ ID NO: 3 ou tout variant allélique ou polymorphique de celle-ci ainsi que les séquences orthologues présentes dans d'autres espèces. Le gène CCR5 code la protéine CCR5 pouvant avoir la séquence représentée par SEO ID NO : 4 ou n'importe quel variant naturel de celle-ci.

CCR3 est également connu sous les noms de CC-CKR-3, CKR-3 et CMKBR3. Le gène CCR3 désigne ici préférentiellement la séquence du gène CCR3 humain dont la séquence d'ARNm peut, par exemple, être la SEQ ID NO : 5 ou tout variant allélique ou polymorphique de celle-ci ainsi que les séquences orthologues présentes dans d'autres espèces. Le gène CCR3 code la protéine CCR3 pouvant avoir la séquence représentée par SEQ ID NO : 6 ou n'importe quel variant naturel de celle-ci.

CCR2 est également connu sous les noms de CCR2b et CMKBR2. Le gène CCR2 désigne ici préférentiellement la séquence du gène CCR2 humain dont la séquence d'ARNm peut, par exemple, être la SEQ ID NO: 7 ou tout variant allélique ou polymorphique de celle-ci ainsi que les séquences orthologues présentes dans d'autres espèces. Le gène CCR2 code la protéine CCR2 pouvant avoir la séquence représentée par SEQ ID NO : 8 ou n'importe quel variant naturel de celle-ci.

CCR1 est également connu sous les noms de CKR1 et CMKBR1. Le gène CCR11 désigne ici préférentiellement la séquence du gène CCR1 humain dont la séquence d'ARNm peut, par exemple, être la SEQ ID NO: 9 ou tout variant allélique ou polymorphique de celle-ci ainsi que les séquences orthologues présentes dans d'autres espèces. Le gène CCR1 code la protéine CCR1 pouvant avoir la séquence représentée par SEQ ID NO : 10 ou n'importe quel variant naturel de celle-ci.

CCR4 est également connu sous le nom de CKR-4. Le gène CCR4 désigne ici préférentiellement la séquence du gène CCR4 humain dont la séquence d'ARNm peut par exemple être la SEQ ID NO : 11 ou tout variant allélique ou polymorphique de celle-ci ainsi que les séquences orthologues présentes dans d'autres espèces. Le gène CCR4 code la protéine CCR4 pouvant être de séquence représentée par SEQ ID NO : 12 ou n'importe quel variant naturel de celle-ci.

CCR8 est également connu sous les noms de ChemR1, TER1 et CMKBR8. Le gène CCR8 désigne ici préférentiellement la séquence du gène CCR8 humain dont la séquence d'ARNm peut, par exemple, être la SEQ ID NO : 13 ou tout variant allélique ou polymorphique de celle-ci ainsi que les séquences orthologues présentes dans d'autres espèces. Le gène CCR8 code la protéine CCR8 pouvant avoir la séquence représentée par SEQ ID NO : 14 ou n'importe quel variant naturel de celle-ci.

CCR9 est également connu sous le nom de D6. Le gène CCR9 désigne ici préférentiellement la séquence du gène CCR9 humain dont la séquence d'ARNm peut, par exemple, être la SEQ ID NO : 15 ou tout variant allélique ou polymorphique de celle-ci ainsi que les séquences orthologues présentes dans d'autres espèces. Le gène CCR9 code la protéine CCR9 pouvant avoir la séquence représentée par SEQ ID NO : 16 ou n'importe quel variant naturel de celle-ci.

CXCR2 est également connu sous le nom de IL-8RB. Le gène CXCR2 désigne ici préférentiellement la séquence du gène CXCR2 humain dont la séquence d'ARNm peut, par exemple, être la SEQ ID NO : 17 ou tout variant allélique ou polymorphique de celle-ci ainsi que les séquences orthologues présentes dans d'autres espèces. Le gène CXCR2 code la protéine CXCR2 pouvant avoir la séquence représentée par SEQ ID NO : 18 ou n'importe quel variant naturel de celle-ci.

STRL33 est également connu sous les noms de Bonzo, CXCR6 et TYMSTR. Le gène STRL33 désigne ici préférentiellement la séquence du gène STRL33 humain dont la séquence d'ARNm peut, par exemple, être la SEQ ID NO : 19 ou tout variant allélique ou polymorphique de celle-ci ainsi que les séquences orthologues présentes dans d'autres espèces. Le gène STRL33 code la protéine STRL33 pouvant avoir la séquence représentée par SEQ ID NO : 20 ou n'importe quel variant naturel de celle-ci.

V28 est également connu sous les noms de CMKBRL1, CX3CR1 et GPR13. Le gène V28 désigne ici préférentiellement la séquence du gène V28 humain dont la séquence d'ARNm peut, par exemple, être la SEQ ID NO : 21 ou tout variant allélique ou polymorphique de celle-ci ainsi que les séquences orthologues présentes dans d'autres espèces. Le gène V28 code la protéine V28 pouvant avoir la séquence représentée par SEQ ID NO : 22 ou n'importe quel variant naturel de celle-ci.

Le gène gpr1 ou GPR1 désigne ici préférentiellement la séquence du gène gpr1 humain dont la séquence d'ARNm peut, par exemple, être la SEQ ID NO : 23 ou tout variant allélique ou polymorphique de celle-ci ainsi que les séquences orthologues présentes dans d'autres espèces. Le gène gpr1 code la protéine gpr1 pouvant avoir la séquence représentée par SEQ ID NO : 24 ou n'importe quel variant naturel de celle-ci.

gpr15 ou GPR15 est également connu sous le nom de BOB. Le gène gpr15 désigne ici préférentiellement la séquence du gène gpr15 humain dont la séquence d'ARNm peut, par exemple, être la SEQ ID NO: 25 ou tout variant allélique ou polymorphique de celle-ci ainsi que les séquences orthologues présentes dans d'autres espèces. Le gène gpr15 code la protéine gpr15 pouvant avoir la séquence représentée par SEQ ID NO : 26 ou n'importe quel variant naturel de celle-ci.

Apj est également connu sous les noms de angiotensin-receptor-like, apelin receptor (APLNR) et AGTRL1. Le gène Apj désigne ici préférentiellement la séquence du gène Apj humain dont la séquence d'ARNm peut, par exemple, être la SEQ ID NO : 27 ou tout variant allélique ou polymorphique de celle-ci ainsi que les séquences orthologues présentes dans d'autres espèces. Le gène Apj code la protéine Apj pouvant avoir la séquence représentée par SEQ ID NO : 28 ou n'importe quel variant naturel de celle-ci.

Le gène ChemR23 est également connu sous les noms de CMKLR1 et DEZ. Le gène ChemR23 désigne ici préférentiellement la séquence du gène ChemR23 humain dont la séquence d'ARNm peut, par exemple, être la SEQ ID NO : 29 ou tout variant allélique ou polymorphique de celle-ci ainsi que les séquences orthologues présentes dans d'autres espèces. Le gène ChemR23 code la protéine ChemR23 pouvant avoir la séquence représentée par SEQ ID NO : 30 ou n'importe quel variant naturel de celle-ci.

Selon l'invention, on entend par « cellule test » toute cellule susceptible d'être infectée par un virus selon l'invention. Préférentiellement, quand le virus est le VIH, la cellule test selon l'invention exprime CD4, un premier et au moins un autre co-récepteur du virus VIH tel que défini ci-dessus. Encore préférentiellement, la cellule test selon l'invention exprime CXCR4 et CCR5. Une cellule test selon l'invention peut exprimer naturellement ces récepteurs ou être génétiquement modifiée afin d'exprimer ces récepteurs. La cellule test selon l'invention peut par exemple être une cellule dendritique, une cellule issue des lignées lymphoïde (préférentiellement un lymphocyte T) ou myéloïde (préférentiellement un macrophage), une cellule épithéliale ou un fibroblaste. Préférentiellement, la cellule test selon l'invention est sélectionnée dans le groupe comprenant les cellules Jurkat (notamment décrites dans Schneider et al. Int J Cancer (1997) 19(5) : 621-6), par exemple le clone de cellule Jurkat E6-1 (ATCC No : TIB-152), les cellules Jurkat-CCR5 (notamment décrites dans Alkhatib et al. (1996) Science 272 : 1955-1958 et AIDS reagent NIBSC, UK). Encore plus préférentiellement, la cellule test selon l'invention est une cellule Jurkat-CCR5.

Les techniques permettant d'infecter une cellule test selon l'invention par un virus VIH sont bien connues de l'homme du métier et sont notamment décrites dans Barré-Sinoussi et al. ((1983) Science 220(4599) : 868-71)).

Le niveau d'expression de microARN ou le niveau d'expression d'ARNm cible dans les cellules test peut être mesuré par toutes techniques connues de l'homme du métier. De nombreuses méthodes sont connues qui permettent de quantifier les ARN, par exemple, les méthodes fondées sur des PCR après transcription inverse (RT-PCR) utilisant des oligonucléotides spécifiques des séquences d'ARN ou des méthodes permettant l'hybridation de ces ARN, des duplicats ou triplicats de ces ARN avec des sondes sous conditions stringentes. Lorsque le niveau d'expression des ARNm cibles est mesuré, il est possible de réaliser une RT-PCR ou des puces cDNA spécifiques avec une seule sonde permettant la transcription inverse de toutes les ARNm. Les sondes selon l'invention sont préférentiellement déposées sur des microarrays. Les conditions stringentes peuvent facilement être déterminées par l'homme du métier. Par exemple, les conditions stringentes selon l'invention peuvent comprendre une étape d'hybridation de 10 à 20 heures, préférablement 16h, à une température de 40 à 50 °C, préférablement à 50°C, en présence d'une force ionique équivalente à celle induite par une concentration de 500mM à 2M de NaCl, préférablement 1 M de NaCl. D'autres produits peuvent également être ajoutés comme des solutions tampon, telles le Tris ou MES, de l'EDTA, du Tween et du BSA (sérum albumine bovine).

Les niveaux d'expression des microARN ou de leurs ARNm cibles ainsi mesurés, dans une cellule test respectivement infectée par un premier virus utilisant un premier co-récepteur et par au moins un autre virus utilisant un autre co-récepteurs, peuvent permettre d'identifier les microARN ou leurs ARNm cibles dont l'expression est modulée lors de l'infection par chacun des virus par rapport à des cellules non infectées. L'identification des microARN ou de leurs ARNm cibles dont l'expression est modulée lors de l'infection par chacun de virus peut être réalisée en comparant les niveaux d'expression des miRNA ou de leurs ARNm cibles mesurés après infection par chacun des virus avec le niveau d'expression desdits miRNA ou desdits ARNm cibles dans des cellules non infectées. Préférentiellement, pour qu'un microARN ou ses ARNm cibles soient considérés comme ayant une expression modulée lors de l'infection des cellules tests par un virus, cette expression est augmentée ou diminuée par rapport à l'expression dans les cellules tests non infectées d'un log2 du rapport (expression dudit miRNA dans des cellules infectées/ expression dudit miRNA dans des cellules non infectées) supérieure à 0,5 ou inférieur à -0,5 respectivement.

Les microARN ou leurs ARNm cibles identifiés comme ayant une expression modulée lors de l'infection par chacun des virus peuvent ensuite être comparés afin de sélectionner les miRNA ou leurs ARNm cibles dont l'expression est modifiée spécifiquement par l'utilisation d'un co-récepteur par le virus.

Par «modification du niveau d'expression spécifique de l'utilisation d'un co-récepteur » selon l'invention on entend une modification, augmentation ou diminution, de l'expression suffisante pour permettre d'identifier le co-récepteur utilisé par le virus.

Par « cellules tests non infectées » ou « cellules non infectées par le virus du patient », on entend des cellules n'ayant pas été mises en contact avec un virus quelque qu'il soit mais également les cellules infectées par un virus, notamment un rétrovirus, dont les co-récepteurs cellulaires sont présentés par les cellules test mais sont distincts du premier co-récepteur ou du au moins un autre co-récepteur utilisé par le virus pour entrer dans la cellule test dans les méthodes selon l'invention. Par exemple, ce virus peut être un virus VIH pseudotypé par une enveloppe amphotrope type VSV ou le virus PFV-1.

Par exemple, si l'expression d'un miRNA ou d'un de ses ARNm cibles est augmentée lors d'une infection avec un premier virus VIH utilisant le co-récepteur CXCR4 dans des cellules Jurkat-CCR5 (exprimant CXCR4 et CCR5) par rapport à l'expression du miRNA ou d'un de ses ARNm cibles dans des cellules Jurkat-CCR5 non infectées et que l'expression de ce miRNA ou d'un de ses ARNm cibles n'est pas augmentée lors d'une infection avec un deuxième virus VIH utilisant le co-récepteur CCR5 dans des cellules Jurkat-CCR5 par rapport à l'expression du miRNA ou d'un de ses ARNm cibles dans les cellules Jurkat-CCR5 non infectées, alors l'augmentation de l'expression dudit miRNA ou dudit ARNm cible est spécifique de l'utilisation du récepteur CXCR4 par le virus VIH.

La présente invention peut concerner également une méthode *in vitro* d'identification de microARN ou de leurs ARNm cibles dont l'expression, lors de l'infection de cellules par un virus utilisant un récepteur et au moins un co-récepteur cellulaire pour entrer dans la cellule, est spécifiquement modifiée en fonction du co-récepteur cellulaire utilisé par le virus pour son entrée dans les cellules, comprenant :
i) déterminer les niveaux d'expression de microARN dans une cellule test, exprimant le récepteur, un premier co-récepteur et au moins un autre co-récepteur, après infection respectivement par un premier virus utilisant le premier co-récepteur et par au moins un autre virus utilisant un autre co-récepteur;
ii) comparer les niveaux d'expression des micro-ARN ainsi déterminés ;
iii) identifier les microARN dont la modification du niveau d'expression est spécifique de l'utilisation d'un co-récepteur.

Les niveaux d'expression des microARN ou de leurs ARNm cibles ainsi mesurés dans une cellule test après infection respectivement par un virus utilisant un premier co-récepteur et au moins un autre virus utilisant un autre co-récepteur peuvent alors être comparés directement entre eux. Cette comparaison permet alors d'identifier les microARN ou leur ARNm cibles dont la modification de l'expression est spécifique de l'utilisation du premier corécepteur ou du au moins un autre co-récepteur par le virus.

Les méthodes selon l'invention peuvent également comprendre un étape supplémentaire permettant d'identifier les ARNm cibles des micro-ARN caractéristiques ainsi identifiés dont la modification du niveau d'expression est spécifique de l'utilisation d'un co-récepteur par un virus pour entrer dans une cellule. Les cibles des miRNA peuvent être identifiées dans des bases de données, notamment miRBase (http://microrna.sanger.ac.uk/ et notamment décrite dans Griffiths-Jones et al (2008) Nucleic Acids Res 36, Griffiths-Jones et al. (2006) Nucleic Acids Res 34, Griffiths-Jones et al. (2004) Nucleic Acids Research 32) et TargetScan (http://www.targetscan.org/ et notamment décrite dans Lewis et al. (2005) Cell 120: 15-20, Grimson et al. (2007) Molecular Cell 27: 91-105, Friedman et al. (2009) Genome Research 19: 92-105).

L'expression « échantillon du patient susceptible de comprendre le virus VIH » comprend tous les liquides ou tissus biologiques provenant d'un patient et pouvant contenir des virus comme par exemple, le sang périphérique, les muqueuses génitales, les tissus lymphoïdes, le liquide céphalo-rachidien, le placenta ou le lait maternel. L'échantillon peut être mis directement en contact avec les cellules test. Préférentiellement, les virus sont extraits de l'échantillon avant contact avec les cellules hôtes. Par exemple, les virus des patients peuvent provenir d'isolats primaires issus d'un échantillon biologique et être obtenus par toutes méthodes connues de l'homme du métier, par exemple, l'isolement des virus VIH peut s'effectuer par co-culture des lymphocytes de patients infectées par le VIH avec des lymphocytes de donneurs séronégatifs pour le VIH notamment selon la technique décrite par Barre-Sinoussi et al ((1983) Science 220(4599):868-71). Le sang périphérique d'un patient infecté par le virus VIH peut également être traité de manière à séparer le plasma des cellules (comme cela est notamment décrit par Fang et al. (1995) Proc. Natl. Acad. Sci. USA 92:12110-4).

Afin de mettre en oeuvre la méthode d'identification d'un co-récepteur cellulaire utilisé par un virus utilisant un récepteur cellulaire et au moins un co-récepteur cellulaire pour entrer dans une cellule chez un patient, le niveau d'expression d'au moins un miRNA et/ou d'au moins un ARNm cible de ce miRNA est mesuré. De préférence ce miRNA et/ou son ARNm cible a été identifié comme ayant une modification du niveau d'expression spécifique de l'utilisation par le virus d'un co-récepteur. De préférence ce miRNA et/ou son ARNm cible aura été identifié par une méthode selon l'invention.

En particulier, la méthode d'identification de virus chez un patient selon l'invention peut être utilisée pour identifier des virus utilisant CXCR4 et/ou CCR5. De préférence ce miRNA et/ou son ARNm cible aura alors été identifié comme ayant une modification du niveau d'expression spécifique de l'utilisation par le virus VIH du récepteur CXCR4 et/ou CCR5. De préférence ce miRNA et/ou son ARNm cible aura été identifié par la méthode d'identification de miRNA selon l'invention.

Préférentiellement, la méthode selon l'invention est mise en oeuvre pour identifier la présence ou l'absence de virus utilisant le co-récepteur CXCR4 chez un patient. La méthode selon l'invention peut aussi être réalisée à plusieurs reprises sur des échantillons issus d'un même patient prélevés à différents moments au cours du temps afin d'identifier l'apparition de virus utilisant le récepteur CXCR4 et ainsi de suivre l'évolution de la maladie chez ce patient.

La valeur prédéterminée peut être une valeur unique comme, par exemple, un niveau ou une moyenne de niveaux d'expression d'un miRNA donné ou d'un ARNm cible donné.

Par exemple, afin d'identifier un virus utilisant un co-récepteur donnée, la valeur prédéterminée peut être la valeur de l'expression d'un miRNA donné ou d'un ARNm cible donné dans une cellule exprimant ce co-récepteur et infectée par un virus de référence connu pour utiliser ce co-récepteur pour entrer dans la cellule. Par exemple, afin d'identifier des virus utilisant CXCR4 et/ou CCR5 chez un patient, la valeur prédéterminée peut être la valeur de l'expression d'un miRNA donné ou d'un ARNm cible donné dans une cellule exprimant CXCR4 ou CCR5 après infection avec un virus VIH de référence utilisant CXCR4 ou CCR5 pour entrer dans une cellule.

La comparaison entre la valeur d'expression obtenue et la valeur prédéterminée permet de déterminer si le virus étudié utilise ou non le même co-récepteur que le virus de référence pour entrer dans les cellules. Par exemple, si la valeur obtenue est proche de la valeur prédéterminée, il est possible de déduire qu'un co-récepteur utilisé par le virus testé est identique à celui utilisé par le virus de référence pour entrer dans les cellules. Par valeur proche on entend préférentiellement des valeurs ne différant pas de plus de 50%, 40%, 30%, 20% ou 10% et encore plus préférentiellement de moins de 5%.

La valeur de référence peut également être, par exemple, la valeur de l'expression d'un miRNA donné ou d'un ARNm cible donné dans une cellule non infectée et donc en absence d'infection par le virus du patient. De préférence, il aura alors été préalablement montré que l'expression dudit miRNA ou dudit ARNm cible est modulée, augmentée ou diminuée, de façon spécifique après infection de la cellule avec un virus de référence utilisant un co-récepteur donné par rapport à l'expression dans une cellule non infectée. L'augmentation ou la diminution de la valeur d'expression d'un miRNA ou d'un ARNm cible de même nature que celle préalablement déterminée indique alors une utilisation d'un même co-récepteur par les virus. Par expression augmentée ou diminuée de même nature, on entend préférentiellement des valeurs de log2 du rapport (expression dudit miRNA et/ou d'un ARNm cible dans des cellules infectées/ expression dudit miRNA et/ou d'un ARNm cible dans des cellules non infectées) de même signe (négative ou positive respectivement). L'étape de comparaison du niveau d'expression à une valeur déterminée (iii) dans la méthode d'identification d'un co-récepteur utilisé par un virus d'un patient infecté selon l'invention est alors préférentiellement mise en oeuvre en déterminant si le niveau d'expression d'au moins un miRNA et/ou au moins un ARNm cible d'un miRNA est augmenté ou diminué par rapport au niveau d'expression du au moins un miRNA et/ou du au moins un ARNm cible d'un miRNA dans des cellules tests non infectées.

Par exemple, la mesure de l'expression d'un ou plusieurs microARN ou ARNm cible, dont l'expression a été montrée comme étant spécifiquement modifiée (augmentée ou diminuée) par des virus VIH de référence utilisant le co-récepteur CXCR4, dans des cellules non infectées peut être comparée a des mesures de l'expression desdits micro-ARN ou ARNm cibles dans des cellules après infection par des virus d'un patient. En cas d'absence de modulations prédéfinies de certains microARN, ce test identifiera que CXCR4 n'est pas un co-récepteur utilisé par un virus VIH du patient. Inversement, si les modulations prédéfinies (augmentation ou diminution) sont observées, le test identifiera que CXCR4 est un co-récepteur utilisé par le virus VIH du patient, il peut être noté qu'un tel virus peut être un virus à double tropisme par exemple, pouvant utiliser CCR5 et CXCR4.

Le miRNA dont l'expression est déterminée peut être, par exemple, sélectionné dans le groupe constitué de hsa-miR-574-5p (notamment de SEQ ID NO : 31, ugagugugugugugugagugugu), hsa-miR-663 (notamment de SEQ ID NO: 32, aggcggggcgccgcgggaccgc), hsa-miR-149* (notamment de SEQ ID NO: 33, agggagggacgggggcugugc), hsa-miR-575 (notamment de SEQ ID NO: 34, gagccaguuggacaggagc), hsa-miR-638 (notamment de SEQ ID NO: 35, agggaucgcgggcggguggcggccu), hsa-miR-181b (notamment de SEQ ID NO: 36, aacauucauugcugucggugggu), hsa-let-7g (notamment de SEQ ID NO: 37, ugagguaguaguuuguacaguu), hsa-miR-30a (notamment de SEQ ID NO: 38, uguaaacauccucgacuggaag), hsa-miR-148a (notamment de SEQ ID NO: 39, ucagugcacuacagaacuuugu) et hsa-miR-9* (notamment de SEQ ID NO: 40, auaaagcuagauaaccgaaagu). Préférentiellement, le mi-RNA dont l'expression est déterminée est hsa-miR-638.

Les miRNA dont l'expression est déterminée peuvent également être des variants allélique ou polymorphique des SEQ ID NO: 31 à 40 ainsi que des séquences orthologues présentes dans d'autres espèces dérivant des miRNA de séquence SEQ ID NO : 31 à 40 et remplissant la même fonction, en particulier régulant l'expression des même ARNm cibles. Par exemple, ces miRNA peuvent dériver des miRNA de séquence SEQ ID NO : 31 à 40 par une ou plusieurs mutations d'acides nucléiques.

L'ARNm dont l'expression est déterminée peut être, par exemple, sélectionné dans le groupe constitué des ARNm cibles des miRNA de SEQ ID NO : 31 à 40 ou des miRNA en dérivant. En particulier, les ARNm cibles peuvent être identifiés dans des bases de données comme décrit ci-dessus.

Par exemple, une augmentation de l'expression d'au moins un miRNA sélectionné dans le groupe comprenant hsa-miR574-5p, hsa-miR-663, hsa-miR-149*, hsa-miR-575, hsa-miR-638 ou une diminution de l'expression d'au moins un microARN sélectionné dans le groupe comprenant hsa-miR-181b, hsa-let-7g, hsa-miR-30a, hsa-miR-148a et hsa-miR-9* indique que CXCR4 est un co-récepteur utilisé par un virus VIH du patient. Au contraire, une absence d'augmentation de l'expression d'au moins un miRNA sélectionné dans le groupe comprenant hsa-miR574-5p, hsa-miR-663, hsa-miR-149*, hsa-miR-575, hsa-miR-638 ou une absence de diminution de l'expression d'au moins un microARN sélectionné dans le groupe comprenant hsa-miR-181b, hsa-let-7g, hsa-miR-30a, hsa-miR-148a et hsa-miR-9* indique que CXCR4 n'est pas un co-récepteur utilisé par un virus du patient.

### FIGURE

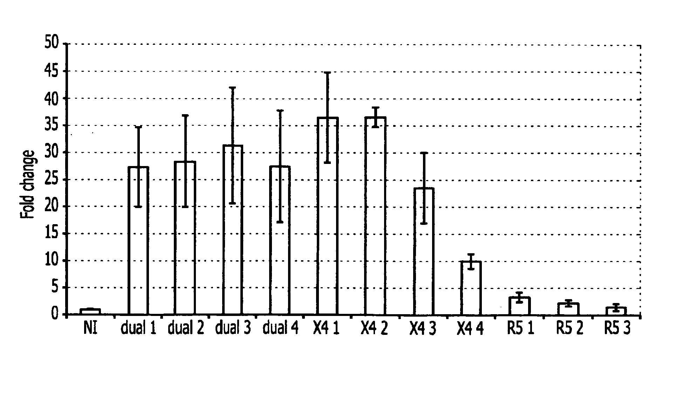

FIG. 1 : **Expression de hsa-miR-638 en réponse à l'infection par des isolats primaires HIV-1 de tropisme CXCR4, CCR5 ou des virus à double tropisme (dual).** Des cellules Jurkat-CCR5 sont infectées par 4 isolats DUAL (dual 1, dual 2, dual 3, dual 4), 4 isolats CXCR4 (X4 1, X4 2, X4 3, X4 4) et 3 isolats CCR5 (R5 1, R5 2, R5 3). Trois jours post-infection, les cellules sont lysées et les ARN sont analysés par RT-qPCR dirigée contre le hsa-miR-638. L'expression de hsa-miR-638 dans les cellules infectées est normalisée par l'expression de cellules contrôles Jurkat R5 non infectées (NI).

### EXEMPLES

### MATERIEL ET METHODES

### Virus et lignées cellulaires

Les virus utilisés dans cette étude sont les virus prototypes VIH-1 NL4.3 et VIH-2 ROD utilisant tous les deux le co-récepteur CXCR4, le virus VIH-1 NLAD8 utilisant le co-récepteur CCR5 et des virus issus d'isolats primaires utilisant le co-récepteur CXCR4 (3 isolats nommés X4 1, X4 2, X4 3 et X4 4), utilisant le co-récepteur CCR5 (3 isolats nommés R5 1, R5 2 et R5 3) ou pouvant utiliser les deux co-récepteurs CXCR4 et CCR5 (4 isolats nommés dual 1, dual 2, dual 3 et dual 4) ainsi qu' un autre rétrovirus, le PFV-1, n'utilisant ni CD4, ni CXCR4 ni CCR5 pour son entrée.

Les lignées cellulaires Jurkat et Jurkat-CCR5 exprimant CCR5 de manière stable sont utilisées.

### Infection

### - infection par les virus prototypes

Les cellules Jurkat et Jurkat-CCR5 sont infectées pendant 3 jours par deux doses infectieuses de VIH-1 NL4.3, VIH-2 ROD afin de tenir compte des modulations liées à la multiplicité d'infection. Les cellules Jurkat-CCR5 sont infectées pendant 3 jours par VIH-1 NLAD8 ou PFV-1.

### - infection par les virus issus d'isolats primaires

Les cellules Jurkat-CCR5 sont infectées pendant 3 jours par les virus X4 1, X4 2, X4 3 et X4 4, R5 1, R5 2, R5 3, dual 1, dual 2, dual 3 ou dual 4. Des cellules Jurkat-CCR5 non infectées sont utilisées en tant que témoin (NI).

### Analyse de l'expression des microARN

### - Analyse par puce micro-ARN

Trois jours après infection par les virus prototypes, les ARN sont extraits et soumis à analyse par puces microARN (LC Sciences ou Affymetrix).

### - Analyse de l'expression de hsa-miR-638 par RT-PCR

Trois jours après infection par les virus issus d'isolats primaires, les cellules sont lysées et l'expression de hsa-miR-368 est analysée par RT-qPCR. L'expression de hsa-miR-368 dans les cellules infectées est normalisée par rapport à l'expression de hsa-miR-368 dans des cellules contrôles Jurkat-CCR5 non infectées (NI).

### RESULTATS

### Exemple 1 : identification des micro-ARN

Les modulations de l'expression des microARN induites par les virus prototypes VIH-1 NL4.3 et VIH-2 ROD, utilisant tous deux le co-récepteur CXCR4, ont été étudiées. Afin de limiter les variations inter-individus et de travailler avec un fond génétique identique (et donc un répertoire de microARN comparable), ces modulations sont étudiées à la fois au cours de l'infection de la lignée cellulaire Jurkat et de la lignée Jurkat exprimant CCR5. Trois jours après l'infection, les ARN des cellules Jurkat sont extraits et soumis à analyse par puces microARN. Le **Tableau 1** met en évidence une sous-population de microARN modulés à la fois par VIH-1 NL4.3 et VIH-2 ROD.

**Tableau 1. Modulations significatives (p<0,01) du répertoire de microARN cellulaires induites lors d'une infection par VIH-1 NL4.3 et VIH-2 ROD de cellules Jurkat et Jurkat-CCR5 à 1 et 100 TCID50**

| **microARN dont l'expression est augmentée lors de l'infection par NL4.3 et ROD** | |
|---|---|
| hsa-miR-574-5p | hsa-miR-575 |
| hsa-miR-663 | hsa-miR-638 |
| hsa-miR-149* | |
| | |

| **microARN dont l'expression est diminuée lors de l'infection par NL4.3 et ROD** | |
|---|---|
| hsa-miR-181b | hsa-miR-374b |
| hsa-let-7g | hsa-miR-148a |
| hsa-miR-26b | hsa-miR-181d |
| hsa-let-7c | hsa-miR-9* |
| hsa-miR-7 | hsa-miR-98 |
| hsa-miR-30a | hsa-let-7e |
| hsa-miR-9 | |

L'infection de cellules Jurkat-CCR5 par VIH-1 NLAD8 (de tropisme R5, Tableau 2) et par le rétrovirus PFV-1 témoin n'utilisant ni CD4, ni CXCR4 ni CCR5 pour son entrée entraîne également des modulations de l'expression de microARN.

**Tableau 2. Modulations significatives (p<0,01) du répertoire de microARN cellulaires induites lors de l'infection VIH-1 NLAD8.**

| **microARN dont l'expression est augmentée lors de l'infection par NLAD8** | |
|---|---|
| hsa-miR-19a | hsa-miR-30b |
| hsa-miR-19b | hsa-miR-23b |
| hsa-miR-30e | hsa-miR-128 |
| hsa-miR-29a | hsa-miR-106a |
| hsa-miR-29c | hsa-miR-15a |
| hsa-miR-342-3p | hsa-miR-17 |
| hsa-miR-30c | hsa-miR-222 |
| hsa-miR-92b | hsa-miR-30d |
| hsa-miR-1280 | hsa-miR-93 |
| hsa-miR-16 | hsa-miR-150 |
| hsa-miR-18b | hsa-let-7i |
| hsa-miR-92a | hsa-miR-25 |
| hsa-miR-18a | hsa-miR-20b |
| hsa-miR-106b | hsa-let-7g |
| hsa-miR-23a | hsa-miR-191 |
| hsa-miR-20a | |
| | |

| **microARN dont l'expression est diminuée lors de l'infection par NLAD8** | |
|---|---|
| hsa-let-7d | hsa-miR-923 |
| hsa-let-7a | hsa-miR-374b |
| hsa-miR-181b | hsa-miR-342-5p |
| hsa-miR-21 | hsa-miR-181d |
| hsa-miR-155 | hsa-miR-638 |
| hsa-miR-26b | hsa-let-7b |
| hsa-miR-1826 | hsa-miR-9 |
| hsa-miR-423-5p | hsa-miR-575 |
| hsa-miR-7 | hsa-miR-1246 |
| hsa-miR-320c | hsa-miR-98 |
| hsa-miR-130b | hsa-miR-149* |
| hsa-miR-182 | hsa-let-7e |
| hsa-miR-320b | hsa-miR-574-5p |
| hsa-miR-320d | hsa-miR-483-5p |
| hsa-let-7c | hsa-miR-375 |
| hsa-miR-1275 | hsa-miR-936 |
| hsa-miR-320a | |

Par comparaison de ces tableaux, on peut constater que l'expression de neuf microARN est systématiquement diminuée lors de l'infection, quelque soit le VIH et son tropisme (Tableau 3). L'expression de ces microARN n'est pas affectée par l'infection par PFV-1.

**Tableau 3. Modulations significatives (p<0,01) du répertoire de microARN cellulaires induites lors de l'infection par NL4.3, ROD et NLAD8.**

| **microARN dont l'expression est diminuée lors de l'infection par NL4.3, ROD et NLAD8** | |
|---|---|
| hsa-miR-181b | hsa-miR-374b |
| hsa-miR-26b | hsa-miR-181d |
| hsa-let-7c | hsa-miR-98 |
| hsa-miR-7 | hsa-let-7e |
| hsa-miR-9 | |

Par ailleurs, l'expression de 5 microARN est augmentée lors de l'infection par NL4.3 ou ROD mais n'est pas augmentée lors de l'infection par NLAD8. Enfin, l'expression de 4 microARN est spécifiquement diminuée lors de l'infection par NL4.3 ou ROD mais n'est pas diminuée lors de l'infection par NLAD8 (Tableau 4). Aucun microARN dont l'expression est augmentée ou diminuée spécifiquement lors de l'infection par NL4.3 ou ROD n'est affecté lors de l'infection par PFV-1.

**Tableau 4. Modulations significatives (p<0,01) du répertoire de microARN cellulaires induites spécifiquement lors de l'infection par NL4.3 et ROD (Augmentée, microARN dont l'expression est augmentée lors de l'infection, Diminuée : microARN dont l'expression est diminuée lors de l'infection).**

| **nom du microARN** | **infection par NL4.3 ou ROD** | **SEQ ID NO :** |
|---|---|---|
| hsa-miR-574-5p | augmentée | 33 |
| hsa-miR-663 | augmentée | 34 |
| hsa-miR-149* | augmentée | 35 |
| hsa-miR-575 | augmentée | 36 |
| hsa-miR-638 | augmentée | 37 |
| hsa-miR-181b | diminuée | 38 |
| hsa-let-7g | diminuée | 39 |
| hsa-miR-30a | diminuée | 40 |
| hsa-miR-148a | diminuée | 41 |
| hsa-miR-9* | diminuée | 42 |

Ces résultats illustrent l'importance des modulations du répertoire de microARN cellulaires induites par l'entrée du virus. Ces analyses mettent également en évidence la possibilité, grâce aux méthodes selon l'invention, de distinguer l'utilisation d'un certain type de co-récepteur (ici CXCR4 ou CCR5) par le VIH sur la base des changements du répertoire cellulaire de microARN.

### Exemple 2 :

Afin de valider les résultats ainsi obtenus, des cellules Jurkat-CCR5 (exprimant à la fois le récepteur CXCR4 et le récepteur CCR5) sont infectées par des virus issus d'isolats primaires utilisant le co-récepteur CXCR4 (X4 1, X4 2, X4 3 et X4 4), utilisant le co-récepteur CCR5 (R5 1, R5 2 et R5 3) ou pouvant utiliser les deux co-récepteurs CXCR4 et CCR5 (dual 1, dual 2, dual 3 et dual 4).

L'expression de hsa-miR-638 dans les cellules infectées ou non infectées (NI, témoin) est mesurée après 3 jours. Comme attendu et comme indiqué dans la figure 1, l'expression de hsa-miR-638 n'est pas modifiée dans les cellules non infectées. Cette expression n'est pas non plus statistiquement modifiée dans les cellules infectées par des virus utilisant uniquement CCR5 comme co-récepteur pour entrer dans les cellules (R5 1, R5 2 et R5 3) (FIG. 1). Par contre, cette expression est significativement augmentée dans les cellules ayant été infectées par un virus capable d'utiliser le co-récepteur CXCR4 pour entrer dans les cellules (X4 1, X4 2, X4 3, X4 4, dual 1, dual 2, dual 3 et dual 4) (FIG. 1).

Ces résultats indiquent que seule une infection faisant intervenir le co-récepteur CXCR4 induit une augmentation de l'expression de hsa-miR-638.

Ces résultats confirment donc les données obtenues dans l'exemple 1 et prouvent, si besoin était, que le niveau d'expression de ce micro-ARN dans une cellule infectée permet de déterminer le co-récepteur utilisé par le virus pour infecter cette cellule et permet donc d'identifier le tropisme de ce virus.

## Revendications

1. Méthode *in vitro* d'identification de microARN, ou de leurs ARNm cibles, dont l'expression, lors de l'infection de cellules par un virus utilisant un récepteur cellulaire et au moins un co-récepteur cellulaire pour entrer dans la cellule, est spécifiquement modifiée en fonction du co-récepteur cellulaire utilisé par le virus pour son entrée dans les cellules, comprenant :
i) déterminer les niveaux d'expression de microARN dans une cellule test, exprimant un récepteur, un premier co-récepteur et au moins un autre co-récepteur, après infection respectivement par un premier virus utilisant le premier co-récepteur et par au moins un autre virus utilisant un autre co-récepteur ;
ii) identifier les microARN dont le niveau d'expression est modulé lors de l'infection par chacun des virus par rapport au niveau d'expression dans des cellules non infectées ;
iii) comparer les microARN ainsi identifiés ;
iv) sélectionner les microARN dont la modification du niveau d'expression est spécifique de l'utilisation d'un co-récepteur ;
v) éventuellement identifier les ARNm cibles des micro-ARN ainsi sélectionnés.

2. Méthode selon la revendication 1, dans laquelle la cellule test exprime un premier et un deuxième co-récepteurs et est infectée par un premier virus utilisant le premier co-récepteur et un deuxième virus utilisant le deuxième co-récepteur pour entrer dans la cellule.

3. Méthode selon la revendication 1 ou 2, dans laquelle les virus sont des rétrovirus.

4. Méthode selon l'une des revendications 1 à 3, dans laquelle les virus sont des virus VIH.

5. Méthode selon la revendication 4, dans laquelle le co-récepteur utilisé par un des virus VIH est sélectionné dans le groupe constitué de CXCR4, CCR5, CCR3, CCR2, CCR1, CCR4, CCR8, CCR9, CXCR2, STRL33, V28, gpr1, gpr15 et ChemR23.

6. Méthode selon la revendication 5, dans laquelle le co-récepteur utilisé par le premier virus VIH est CXCR4 et le co-récepteur utilisé par le deuxième virus VIH est CCR5.

7. Méthode selon l'une des revendications 4 à 6, dans laquelle les cellules test sont les cellules Jurkat-CCR5.

8. Méthode *in vitro* d'identification d'un co-récepteur cellulaire utilisé par un virus utilisant un récepteur cellulaire et au moins un co-récepteur cellulaire pour entrer dans une cellule, chez un patient infecté par le virus, comprenant :
i) mettre en contact un échantillon du patient susceptible de contenir le virus avec une cellule test exprimant un récepteur cellulaire du virus et au moins un co-récepteur cellulaire du virus ;
ii) déterminer le niveau d'expression d'au moins un miRNA dont l'expression est spécifiquement modifiée en fonction du co-récepteur cellulaire utilisé par le virus pour son entrée dans les cellules et/ou d'au moins un ARNm cible dudit miRNA dans la cellule test ;
iii) comparer le niveau d'expression à une valeur prédéterminée ;
iv) en déduire si le virus utilise ou non un co-récepteur cellulaire exprimé par la cellule test.

9. Méthode selon la revendication 8, dans laquelle la valeur prédéterminée est le niveau d'expression du miRNA ou de l'ARNm dans une cellule test non infectée.

10. Méthode selon la revendication 8 ou 9, dans laquelle le virus est le virus VIH et dans laquelle la cellule test exprime le récepteur cellulaire CD4.

11. Méthode selon la revendication 10, dans laquelle la cellule test exprime un co-récepteur cellulaire sélectionné dans le groupe constitué de CXCR4, CCR5, CCR3, CCR2, CCR1, CCR4, CCR8, CCR9, CXCR2, STRL33, V28, gpr1, gpr15 et ChemR23.

12. Méthode selon la revendication 10, dans laquelle la cellule test exprime CXCR4.

13. Méthode selon l'une des revendication 10 à 12, dans laquelle le microARN est sélectionné dans le groupe constitué de hsa-miR574-5p, hsa-miR-663, hsa-miR-149*, hsa-miR-575, hsa-miR-638, hsa-miR-181b, hsa-let-7g, hsa-miR-30a, hsa-miR-148a et hsa-miR-9*.

14. Méthode selon la revendication 13, dans laquelle une augmentation de l'expression d'au moins un microARN sélectionné dans le groupe comprenant hsa-miR574-5p, hsa-miR-663, hsa-miR-149*, hsa-miR-575, hsa-miR-638 ou une diminution de l'expression d'au moins un microARN sélectionné dans le groupe comprenant hsa-miR-181 b, hsa-let-7g, hsa-miR-30a, hsa-miR-148a et hsa-miR-9* indique que CXCR4 est un co-récepteur utilisé par le virus VIH.

15. Méthode selon l'une des revendications 8 à 14, dans laquelle le niveau d'expression des microARN ou la quantité de mRNA est mesuré(e) par RT-PCR ou à l'aide d'une micropuce.

16. Méthode selon l'une des revendications 10 à 15, dans laquelle les cellules test sont sélectionnées dans le groupe constitué des cellules Jurkat et des cellules Jurkat-CCR5.

## Patentansprüche

1. *in vitro*-Identifikationsmethode von microRNA oder ihrer Ziel-mRNA, deren Expression bei der Infektion von Zellen durch ein Virus, das einen Zellrezeptor und mindestens einen Zell-Corezeptor verwendet, um in die Zelle einzudringen, in Abhängigkeit vom von dem Virus für sein Eindringen in die Zellen verwendeten Zell-Corezeptor speziell modifiziert ist, die umfasst:
i) Bestimmen der Expressionsniveaus von microRNA in einer Testzelle, die einen Rezeptor, einen ersten Corezeptor und mindestens einen anderen Corezeptor exprimiert, nach Infektion jeweils durch ein erstes Virus, das den ersten Corezeptor verwendet, und durch mindestens ein anderes Virus, das einen anderen Corezeptor verwendet,
ii) Identifizieren der microRNA, deren Expressionsniveau bei der Infektion durch jedes der Viren im Verhältnis zum Expressionsniveau in nicht infizierten Zellen moduliert ist,
iii) Vergleichen der derart identifizierten microRNA,
iv) Auswählen der microRNA, deren Modifikation des Expressionsniveaus für die Verwendung eines Corezeptors spezifisch ist,
v) eventuell identifizieren der Ziel-mRNA der derart ausgewählten microRNA.

2. Methode nach Anspruch 1, wobei die Testzelle einen ersten und einen zweiten Corezeptor exprimiert und von einem ersten Virus, das den ersten Corezeptor verwendet, und einem zweiten Virus, das den zweiten Corezeptor verwendet, um in die Zelle einzudringen, infiziert ist.

3. Methode nach Anspruch 1 oder 2, wobei die Viren Retroviren sind.

4. Methode nach einem der Ansprüche 1 bis 3, wobei die Viren HIV-Viren sind.

5. Methode nach Anspruch 4, wobei der von einem der HIV-Viren verwendete Corezeptor aus der Gruppe ausgewählt ist, die von CXCR4, CCR5, CCR3, CCR2, CCR1 , CCR4, CCR8, CCR9, CXCR2, STRL33, V28, gprl, gprl 5 und ChemR23 gebildet ist.

6. Methode nach Anspruch 5, wobei der von dem ersten HIV-Virus verwendete Corezeptor CXCR4 ist und der von dem zweiten HIV-Virus verwendete Corezeptor CCR5 ist.

7. Methode nach einem der Ansprüche 4 bis 6, wobei die Testzellen die Jurkat-CCR5-Zellen sind.

8. *in vitro*-Identifikationsmethode eines Zell-Corezeptors, der von einem Virus verwendet wird, das einen Zellrezeptor und mindestens einen Zell-Corezeptor verwendet, um bei einem von dem Virus infizierten Patienten in eine Zelle einzudringen , die umfasst:
i) Inkontaktversetzen einer Probe des Patienten, die das Virus enthalten könnte, mit einer Testzelle, die einen Zellrezeptor des Virus und mindestens einen Zell-Corezeptor des Virus exprimiert,
ii) Bestimmen des Expressionsniveaus mindestens einer miRNA, deren Expressionsniveau in Abhängigkeit von dem Zell-Corezeptor, der von dem Virus für sein Eindringen in die Zellen verwendet wird, speziell modifiziert ist, und/oder mindestens einer Ziel-mRNA der miRNA in der Testzelle,
iii) Vergleichen des Expressionsniveaus mit einem vorbestimmten Wert,
iv) Ableiten daraus, ob das Virus einen Zell-Corezeptor benutzt, der von der Testzelle exprimiert wird, oder nicht.

9. Methode nach Anspruch 8, wobei der vorbestimmte Wert das Expressionsniveau der miRNA oder der mRNA in einer nicht infizierten Testzelle ist.

10. Methode nach Anspruch 8 oder 9, wobei das Virus das HIV-Virus ist und wobei die Testzelle den Zellrezeptor CD4 exprimiert.

11. Methode nach Anspruch 10, wobei die Testzelle einen Zell-Corezeptor exprimiert, der aus der Gruppe ausgewählt ist, die von CXCR4, CCR5, CCR3, CCR2, CCR1, CCR4, CCR8, CCR9, CXCR2, STRL33, V28, gprl, gpr15 und ChemR23 gebildet ist.

12. Methode nach Anspruch 10, wobei die Testzelle CXCR4 exprimiert.

13. Methode nach einem der Ansprüche 10 bis 12, wobei die microRNA aus der Gruppe ausgewählt ist, die von hsa-miR574-5p, hsa-miR-663, hsa-miR-149*, hsa-miR-575, hsa-miR-638, hsa-miR-181 b, hsa-let-7g, hsa-miR-30a, hsa-miR-148a und hsa-miR-9* gebildet ist.

14. Methode nach Anspruch 13, wobei eine Erhöhung der Expression mindestens einer microRNA, ausgewählt aus der Gruppe umfassend hsa-miR574-5p, hsa-miR-663, hsa-miR-149*, hsa-miR-575, hsa-miR-638, oder eine Verringerung der Expression mindestens einer microRNA, ausgewählt aus der Gruppe unfassend hsa-miR-181b, hsa-let-7g, hsa-miR-30a, hsa-miR-148a und hsa-miR-9*, anzeigt, dass CXCR4 ein von dem HIV-Virus verwendeter Corezeptor ist.

15. Methode nach einem der Ansprüche 8 bis 14, wobei das Expressionsniveau der microRNA oder die Menge von mRNA durch RT-PCR oder mit Hilfe eines Mikrochips gemessen wird.

16. Methode nach einem der Ansprüche 10 bis 15, wobei die Testzellen aus der Gruppe ausgewählt sind, die von den Jurkat-Zellen und den Jurkat-CCR5-Zellen gebildet ist.

## Claims

1. An *in vitro* method for identifying microRNAs, or their target mRNAs, the expression of which, during infection of cells by a virus using a cell receptor and at least one cell co-receptor for entering the cell, is specifically modified according to the cell co-receptor used by the virus for its entry into the cells, comprising:
i) determining the expression levels of microRNAs in a test cell, expressing a receptor, a first co-receptor and at least one other co-receptor, after infection by a first virus using the first co-receptor and by at least one other virus using another co-receptor, respectively;
ii) identifying the microRNAs, the expression level of which is modulated during the infection by each of the viruses relatively to the expression level in uninfected cells;
iii) comparing the thus-identified microRNAs;
iv) selecting the microRNAs, the modification of the expression level of which is specific to the use of a co-receptor;
v) optionally identifying the target mRNAs of the thereby selected microRNAs.

2. The method according to claim 1, wherein the test cell expresses a first and a second co-receptors and is infected with a first virus using the first co-receptor and a second virus using the second co-receptor for entering the cell.

3. The method according to claim 1 or 2, wherein the viruses are retroviruses.

4. The method according to any one of claims 1 to 3, wherein the viruses are HIV viruses.

5. The method according to claim 4, wherein the co-receptor used by one of the HIV viruses is selected from the group consisting of CXCR4, CCR5, CCR3, CCR2, CCR1, CCR4, CCR8, CCR9, CXCR2, STRL33, V28, gpr1, gpr15 and ChemR23.

6. The method according to claim 5, wherein the co-receptor used by the first HIV virus is CXCR4 and the co-receptor used by the second HIV virus is CCR5.

7. The method according to any one of claims 4 to 6, wherein the test cells are Jurkat-CCR5 cells.

8. An *in vitro* method for identifying a cell co-receptor used by a virus using a cell receptor and at least one cell co-receptor for entering a cell, in a patient infected with the virus, comprising:
i) putting a sample of the patient which may contain the virus in contact with a test cell expressing a cell receptor of the virus and at least one cell co-receptor of the virus;
ii) determining the expression level of at least one miRNA, the expression of which is specifically modified depending on the cell co-receptor used by the virus for its entry into the cells and/or of at least one target mRNA of said miRNA in the test cell;
iii) comparing the expression level with a predetermined value;
iv) inferring therefrom whether the virus uses or not a cell co-receptor expressed by the test cell.

9. The method according to claim 8, wherein the predetermined value is the expression level of the miRNA or of the mRNA in an uninfected test cell.

10. The method according to claim 8 or 9, wherein the virus is the HIV virus and wherein the test cell expresses the cell receptor CD4.

11. The method according to claim 10, wherein the test cell expresses a cell co-receptor selected from the group consisting of CXCR4, CCR5, CCR3, CCR2, CCR1, CCR4, CCR8, CCR9, CXCR2, STRL33, V28, gpr1, gpr15 and ChemR23.

12. The method according to claim 10, wherein the test cell expresses CXCR4.

13. The method according to any one of claims 10 to 12, wherein the microRNA is selected from the group consisting of hsa-miR574-5p, hsa-miR-663, hsa-miR-149*, hsa-miR-575, hsa-miR-638, hsa-miR-181b, hsa-let-7g, hsa-miR-30a, hsa-miR-148a and hsa-miR-9*.

14. The method according to claim 13, wherein an increase in the expression of at least one microRNA selected from the group comprising hsa-miR574-5p, hsa-miR-663, hsa-miR-149*, hsa-miR-575, hsa-miR-638 or a decrease in the expression of at least one microRNA selected from the group comprising hsa-miR-181b, hsa-let-7g, hsa-miR-30a, hsa-miR-148a and hsa-miR-9* indicates that CXCR4 is a co-receptor used by the HIV virus.

15. The method according to any one of claims 8 to 14, wherein the expression level of the microRNAs or the amount of mRNA is measured by RT-PCR or by means of a microchip.

16. The method according to any one of claims 10 to 15, wherein the test cells are selected from the group consisting of Jurkat cells and of Jurkat-CCR5 cells.


### SEQUENCE LISTING

<110> Centre National de la Recherche Scientifique (CNRS)
<120> Identification du tropisme cellulaire de virus
<130> BFF09P0281
<160> 40
<170> PatentIn version 3.4
<210> 1
   <211> 4320
   <212> DNA
   <213> Homo sapiens
<220>
   <221> CDS
   <222> (1038)..(1052)
<220>
   <221> CDS
   <222> (3185)..(4225)
<400> 1
   tttcatctct ccgggcttat ttgctggttt ctccgaatgc gggccttgtc tggttcacgc     60
   tggatcccca acgcctagaa cagtgcgtgg cacgcagttc gtccttctat aaatatcgga    120
   ctaaatgcat ctctgtgatg gtaataccca cacggtgttg tgagaatgaa tgagtgattc    180
   tgtgcaagtt cctagtgatc tgttacaaaa agtactggtc gctaaattac tcttataata    240
   aagcatactt ttaggataat aaagcactat tcgcgaattg gttaccgcta ttatgaaatt    300
   actgagcaat acatatctac atctgatcag tctccagaat tatgccaaat cctaccttct    360
   tctgaaagta tctcctaatt atctgcacct gaccctagtg atgctgtgaa tgtgcaagta    420
   tagctacatc ctccgaagga aggatcttta ctccttttac ctcctgaatg ggctgcgtct    480
   gctgaaagcg cgggggaatg ggcggttgga agcttggccc tacttccagc attgccgcct    540
   actggttggg ttactccagc aagtcactcc ccttccctgg gcctcagtgt ctctactgta    600
   gcattcccag gtctggaatt ccatccactt tagcaaggat ggacgcgcca cagagagacg    660
   cgttcctagc ccgcgcttcc cacctgtctt caggcgcatc ccgcttccct caaacttagg    720
   aaatgcctct gggaggtcct gtccggctcc ggactcacta ccgaccaccc gcaaacagca    780
   gggtcccctg ggcttcccaa gccgcgcacc tctccgcccc gcccctgcgc cctccttcct    840
   cgcgtctgcc cctctccccc accccgcctt ctccctcccc gccccagcgg cgcatgcgcc    900
   gcgctcggag cgtgttttta taaaagtccg gccgcggcca gaaacttcag tttgttggct    960
   gcggcagcag gtagcaaagt gacgccgagg gcctgagtgc tccagtagcc accgcatctg   1020
   gagaaccagc ggttacc atg gag ggg atc agt gtaagtccag tttcaacctg       1072
                      Met Glu Gly Ile Ser
                      1               5
   ctttgtcata aatgtacaaa cgtttgaact tagagcgcag cccctctccg agcgggcaga 1132
   agcggccagg acattggagg tacccgtact ccaaaaaagg gtcaccgaaa ggagttttct 1192
   tgaccatgcc tatatagtgc gggtgggtgg ggggggagca ggattggaat ctttttctct 1252
   gtgagtcgag gagaaacgac tggaaagagc gttccagtgg ctgcatgtgt ctcccccttg 1312
   agtcccgccg cgcgcggcgg cttgcacgct gtttgcaaac gtaagaacat tctgtgcaca 1372
   agtgcagaga aggcgtgcgc gctgcctcgg gactcagacc accggtctct tccttgggga 1432
   agcggggatg tcttggagcg agttacattg tctgaattta gaggcggagg gcggcgtgcc 1492
   tgggctgact tcccaggagg agattgcgcc cgctttaact tcggggttaa gcgcctggtg 1552
   actgttcttg acactgggtg cgtgtttgtt aaactctgtg cggccgacgg agctgtgcca 1612
   gtctcccagc acagtaggca gagggcggga gaggcgggtg gacccaccgc gccgatcctc 1672
   tgaggggatc gagtggtggc agcagctagg agttgatccg cccgcgcgct ttgggtttga 1732
   gggggaaacc ttcccgccgt ccgaagcgcg cctcttcccc acggccgcga gtgggtcctg 1792
   cagttcgaga gtttggggtc gtgcagaggt cagcggagtg gtttgacctc ccctttgaca 1852
   ccgcgcagct gccagccctg agatttgcgc tccggggata ggagcgggta cggggtgagg 1912
   ggcgggggcg gttaagaccg cacctgggct gccaggtcgc cgccgcgaag actggcaggt 1972
   gcaagtgggg aaaccgtttg gctctctccg agtccagttg tgatgtttaa ccgtcggtgg 2032
   tttccagaaa ccttttgaaa ccctcttgct agggagtttt tggtttcctg cagcggcgcg 2092
   caattcaaag acgctcgcgg cggagccgcc cagtcgctcc ccagcaccct gtgggacaga 2152
   gcctggcgtg tcgcccagcg gagcccctgc agcgctgctt gcgggcggtt ggcgtgggtg 2212
   tagtgggcag ccgcggcggc ccggggctgg acgacccggc cccccgcgtg cccaccgcct 2272
   ggaggcttcc agctgcccac ctccggccgg gttaactgga tcagtggcgg ggtaatggga 2332
   agccacccgg gagagtgagg aaatgaaact tggggcgagg accacgggtg cagaccccgt 2392
   taccttctcc acccaggaaa atgccccgct ccctaacgtc ccaaacgcgc caagtgataa 2452
   acacgaggat ggcaagagac ccacacaccg gaggagcgcc cgcttggggg aggaggtgcc 2512
   gtttgttcat tttctgacac tcccgcccaa tataccccaa gcaccgaagg gccttcgttt 2572
   taagaccgca ttctctttac ccactacaag ttgcttgaag cccagaatgg tttgtattta 2632
   ggcaggcgtg ggaaaattaa gtttttgcgc tttaggagaa tgagtctttg caacgccccc 2692
   gccctccccc cgtgatcctc ccttctcccc tcttccctcc ctgggcgaaa aacttcttac 2752
   aaaaagttaa tcactgcccc tcctagcagc acccacccca ccccccacgc cgcctgggag 2812
   tggcctcttt gtgtgtattt tttttttcct cctaaggaag gttttttttc ttccctctag 2872
   tgggcggggc agaggagtta gccaagatgt gactttgaaa ccctcagcgt ctcagtgccc 2932
   ttttgttcta aacaaagaat tttgtaattg gttctaccaa agaaggatat aatgaagtca 2992
   ctatgggaaa agatggggag gagagttgta ggattctaca ttaattctct tgtgccctta 3052
   gcccactact tcagaatttc ctgaagaaag caagcctgaa ttggtttttt aaattgcttt   3112
   aaaaattttt tttaactggg ttaatgcttg ctgaattgga agtgaatgtc cattcctttg   3172
   cctcttttgc ag ata tac act tca gat aac tac acc gag gaa atg ggc tca   3223
                 Ile Tyr Thr Ser Asp Asn Tyr Thr Glu Glu Met Gly Ser
                                 10                  15
   ggg gac tat gac tcc atg aag gaa ccc tgt ttc cgt gaa gaa aat gct    3271
   Gly Asp Tyr Asp Ser Met Lys Glu Pro Cys Phe Arg Glu Glu Asn Ala
       20                  25                  30
   aat ttc aat aaa atc ttc ctg ccc acc atc tac tcc atc atc ttc tta    3319
   Asn Phe Asn Lys Ile Phe Leu Pro Thr Ile Tyr Ser Ile Ile Phe Leu
   35                  40                  45                  50
   act ggc att gtg ggc aat gga ttg gtc atc ctg gtc atg ggt tac cag    3367
   Thr Gly Ile Val Gly Asn Gly Leu Val Ile Leu Val Met Gly Tyr Gln
                   55                  60                  65
   aag aaa ctg aga agc atg acg gac aag tac agg ctg cac ctg tca gtg    3415
   Lys Lys Leu Arg Ser Met Thr Asp Lys Tyr Arg Leu His Leu Ser Val
               70                  75                  80
   gcc gac ctc ctc ttt gtc atc acg ctt ccc ttc tgg gca gtt gat gcc    3463
   Ala Asp Leu Leu Phe Val Ile Thr Leu Pro Phe Trp Ala Val Asp Ala
           85                  90                  95
   gtg gca aac tgg tac ttt ggg aac ttc cta tgc aag gca gtc cat gtc    3511
   Val Ala Asn Trp Tyr Phe Gly Asn Phe Leu Cys Lys Ala Val His Val
       100                 105                 110
   atc tac aca gtc aac ctc tac agc agt gtc ctc atc ctg gcc ttc atc    3559
   Ile Tyr Thr Val Asn Leu Tyr Ser Ser Val Leu Ile Leu Ala Phe Ile
   115                 120                 125                 130
   agt ctg gac cgc tac ctg gcc atc gtc cac gcc acc aac agt cag agg    3607
   Ser Leu Asp Arg Tyr Leu Ala Ile Val His Ala Thr Asn Ser Gln Arg
                   135                 140                 145
   cca agg aag ctg ttg gct gaa aag gtg gtc tat gtt ggc gtc tgg atc    3655
   Pro Arg Lys Leu Leu Ala Glu Lys Val Val Tyr Val Gly Val Trp Ile
               150                 155                 160
   cct gcc ctc ctg ctg act att ccc gac ttc atc ttt gcc aac gtc agt    3703
   Pro Ala Leu Leu Leu Thr Ile Pro Asp Phe Ile Phe Ala Asn Val Ser
           165                 170                 175
   gag gca gat gac aga tat atc tgt gac cgc ttc tac ccc aat gac ttg    3751
   Glu Ala Asp Asp Arg Tyr Ile Cys Asp Arg Phe Tyr Pro Asn Asp Leu
       180                 185                 190
   tgg gtg gtt gtg ttc cag ttt cag cac atc atg gtt ggc ctt atc ctg    3799
   Trp Val Val Val Phe Gln Phe Gln His Ile Met Val Gly Leu Ile Leu
   195                 200                 205                 210
   cct ggt att gtc atc ctg tcc tgc tat tgc att atc atc tcc aag ctg    3847
   Pro Gly Ile Val Ile Leu Ser Cys Tyr Cys Ile Ile Ile Ser Lys Leu
                   215                 220                 225
   tca cac tcc aag ggc cac cag aag cgc aag gcc ctc aag acc aca gtc    3895
   Ser His Ser Lys Gly His Gln Lys Arg Lys Ala Leu Lys Thr Thr Val
               230                 235                 240
   atc ctc atc ctg gct ttc ttc gcc tgt tgg ctg cct tac tac att ggg      3943
   Ile Leu Ile Leu Ala Phe Phe Ala Cys Trp Leu Pro Tyr Tyr Ile Gly
           245                     250                     255
   atc agc atc gac tcc ttc atc ctc ctg gaa atc atc aag caa ggg tgt      3991
   Ile Ser Ile Asp Ser Phe Ile Leu Leu Glu Ile Ile Lys Gln Gly Cys
       260                     265                     270
   gag ttt gag aac act gtg cac aag tgg att tcc atc acc gag gcc cta      4039
   Glu Phe Glu Asn Thr Val His Lys Trp Ile Ser Ile Thr Glu Ala Leu
   275                     280                     285                 290
   gct ttc ttc cac tgt tgt ctg aac ccc atc ctc tat gct ttc ctt gga      4087
   Ala Phe Phe His Cys Cys Leu Asn Pro Ile Leu Tyr Ala Phe Leu Gly
                   295                     300                     305
   gcc aaa ttt aaa acc tct gcc cag cac gca ctc acc tct gtg agc aga      4135
   Ala Lys Phe Lys Thr Ser Ala Gln His Ala Leu Thr Ser Val Ser Arg
               310                     315                     320
   ggg tcc agc ctc aag atc ctc tcc aaa gga aag cga ggt gga cat tca      4183
   Gly Ser Ser Leu Lys Ile Leu Ser Lys Gly Lys Arg Gly Gly His Ser
           325                     330                     335
   tct gtt tcc act gag tct gag tct tca agt ttt cac tcc agc              4225
   Ser Val Ser Thr Glu Ser Glu Ser Ser Ser Phe His Ser Ser
       340                     345                     350
   taacacagat gtaaaagact tttttttata cgataaataa ctttttttta agttacacat      4285
   ttttcagata taaaagactg accaatattg tacag                               4320
<210> 2
   <211> 352
   <212> PRT
   <213> Homo sapiens
<400> 2
   Met Glu Gly Ile Ser Ile Tyr Thr Ser Asp Asn Tyr Thr Glu Glu Met
   1               5                   10                  15
   Gly Ser Gly Asp Tyr Asp Ser Met Lys Glu Pro Cys Phe Arg Glu Glu
               20                  25                  30
   Asn Ala Asn Phe Asn Lys Ile Phe Leu Pro Thr Ile Tyr Ser Ile Ile
           35                  40                  45
   Phe Leu Thr Gly Ile Val Gly Asn Gly Leu Val Ile Leu Val Met Gly
       50                  55                  60
   Tyr Gln Lys Lys Leu Arg Ser Met Thr Asp Lys Tyr Arg Leu His Leu
   65                  70                  75                  80
   Ser Val Ala Asp Leu Leu Phe Val Ile Thr Leu Pro Phe Trp Ala Val
                   85                  90                  95
   Asp Ala Val Ala Asn Trp Tyr Phe Gly Asn Phe Leu Cys Lys Ala Val
                   100                 105                 110
   His Val Ile Tyr Thr Val Asn Leu Tyr Ser Ser Val Leu Ile Leu Ala
               115                 120                 125
   Phe Ile Ser Leu Asp Arg Tyr Leu Ala Ile Val His Ala Thr Asn Ser
       130                 135                 140
   Gln Arg Pro Arg Lys Leu Leu Ala Glu Lys Val Val Tyr Val Gly Val
   145                 150                 155                 160
   Trp Ile Pro Ala Leu Leu Leu Thr Ile Pro Asp Phe Ile Phe Ala Asn
                   165                 170                 175
   Val Ser Glu Ala Asp Asp Arg Tyr Ile Cys Asp Arg Phe Tyr Pro Asn
               180                 185                 190
   Asp Leu Trp Val Val Val Phe Gln Phe Gln His Ile Met Val Gly Leu
           195                 200                 205
   Ile Leu Pro Gly Ile Val Ile Leu Ser Cys Tyr Cys Ile Ile Ile Ser
       210                 215                 220
   Lys Leu Ser His Ser Lys Gly His Gln Lys Arg Lys Ala Leu Lys Thr
   225                 230                 235                 240
   Thr Val Ile Leu Ile Leu Ala Phe Phe Ala Cys Trp Leu Pro Tyr Tyr
                   245                 250                 255
   Ile Gly Ile Ser Ile Asp Ser Phe Ile Leu Leu Glu Ile Ile Lys Gln
               260                 265                 270
   Gly Cys Glu Phe Glu Asn Thr Val His Lys Trp Ile Ser Ile Thr Glu
           275                 280                 285
   Ala Leu Ala Phe Phe His Cys Cys Leu Asn Pro Ile Leu Tyr Ala Phe
       290                 295                 300
   Leu Gly Ala Lys Phe Lys Thr Ser Ala Gln His Ala Leu Thr Ser Val
   305                 310                 315                 320
   Ser Arg Gly Ser Ser Leu Lys Ile Leu Ser Lys Gly Lys Arg Gly Gly
                   325                 330                 335
   His Ser Ser Val Ser Thr Glu Ser Glu Ser Ser Ser Phe His Ser Ser
               340                 345                 350
<210> 3
   <211> 1560
   <212> DNA
   <213> Homo sapiens
<220>
   <221> CDS
   <222> (358)..(1416)
<400> 3
   cttcagatag attatatctg gagtgaagaa tcctgccacc tatgtatctg gcatagtatt     60
   ctgtgtagtg ggatgagcag agaacaaaaa caaaataatc cagtgagaaa agcccgtaaa    120
   taaaccttca gaccagagat ctattctcta gcttatttta agctcaactt aaaaagaaga    180
   actgttctct gattcttttc gccttcaata cacttaatga tttaactcca ccctccttca    240
   aaagaaacag catttcctac ttttatactg tctatatgat tgatttgcac agctcatctg    300
   gccagaagag ctgagacatc cgttccccta caagaaactc tccccgggtg gaacaag       357
   atg gat tat caa gtg tca agt cca atc tat gac atc aat tat tat aca      405
   tcg gag ccc tgc caa aaa atc aat gtg aag caa atc gca gcc cgc ctc      453
   ctg cct ccg ctc tac tca ctg gtg ttc atc ttt ggt ttt gtg ggc aac      501
   atg ctg gtc atc ctc atc ctg ata aac tgc aaa agg ctg aag agc atg      549
   act gac atc tac ctg ctc aac ctg gcc atc tct gac ctg ttt ttc ctt      597
   ctt act gtc ccc ttc tgg gct cac tat gct gcc gcc cag tgg gac ttt      645
   gga aat aca atg tgt caa ctc ttg aca ggg ctc tat ttt ata ggc ttc      693
   ttc tct gga atc ttc ttc atc atc ctc ctg aca atc gat agg tac ctg      741
   gct gtc gtc cat gct gtg ttt gct tta aaa gcc agg acg gtc acc ttt      789
   ggg gtg gtg aca agt gtg atc act tgg gtg gtg gct gtg ttt gcg tct      837
   ctc cca gga atc atc ttt acc aga tct caa aaa gaa ggt ctt cat tac      885
   acc tgc agc tct cat ttt cca tac agt cag tat caa ttc tgg aag aat      933
   ttc cag aca tta aag ata gtc atc ttg ggg ctg gtc ctg ccg ctg ctt      981
   gtc atg gtc atc tgc tac tcg gga atc cta aaa act ctg ctt cgg tgt     1029
   cga aat gag aag aag agg cac agg gct gtg agg ctt atc ttc acc atc     1077
   atg att gtt tat ttt ctc ttc tgg gct ccc tac aac att gtc ctt ctc     1125
   ctg aac acc ttc cag gaa ttc ttt ggc ctg aat aat tgc agt agc tct     1173
   aac agg ttg gac caa gct atg cag gtg aca gag act ctt ggg atg acg     1221
   cac tgc tgc atc aac ccc atc atc tat gcc ttt gtc ggg gag aag ttc     1269
   aga aac tac ctc tta gtc ttc ttc caa aag cac att gcc aaa cgc ttc     1317
   tgc aaa tgc tgt tct att ttc cag caa gag gct ccc gag cga gca agc     1365
   tca gtt tac acc cga tcc act ggg gag cag gaa ata tct gtg ggc ttg     1413
   tga cacggactca agtgggctgg tgacccagtc agagttgtgc acatggctta          1466
   gttttcatac acagcctggg ctgggggtgg ggtgggagag gtctttttta aaaggaagtt   1526
   actgttatag agggtctaag attcatccat ttat                               1560
<210> 4
   <211> 352
   <212> PRT
   <213> Homo sapiens
<400> 4
<210> 5
   <211> 5160
   <212> DNA
   <213> Homo sapiens
<220>
   <221> CDS
   <222> (4015)..(5082)
<400> 5
   ttcttcctaa atttatttac aaatgtaaca caattccacc caaacttatg tttttataag      60
   taattgagta gatgatccta aagtttaata aaacaaatgg ctctaatagg taagacattt     120
   ggaaatgtat aatgaaaggg agttgcataa taagatcatc tatataaatc atctaataaa     180
   tctacaataa aaagtgtctc tagcacagaa ataagatatc aatagaatat aaggtacaaa     240
   atcagattca ggaacattaa agaatatacg acaaaggtga tatttcaagc ccaaagggga     300
   gaagatggtt attcaacaca tagtgtttta aaatttgtca gataagaatg gagaggagga     360
   ggctcctctc ctctgacccc agggaatgtg agaagagaca cagtggttat gaaaggaagc     420
   agtcacacct gtggatccct accttcccca tcagagctag ggggcatgga gcgctctctg     480
   ctaagatggg gacccccaag gaatgtctcc ctgtggggca cttccttacc agatgggatg     540
   gccagtgcgg ttaagttggt ggtcaggcag aaaaaaaaga tctagtttgt actcttgaga     600
   gttcctcggt ttgttcatgg catgggcagg gagtcaagga gcagcagcct tgcctcagtg     660
   cctaccagtg caggaaaagg tgcatagcct gggccagggc cagggccctg gtggaggcgt     720
   agtggtaaca gagagggctc tccattccag cccaaggaag actaagaatg aatacctcat     780
   gagtatatta gctacaaacc accacagcag gttccagaaa aaggctcagc gttggaacca     840
   ggtcaccccc actcagcaga caccagtcat ataaatcaag gaccaacagg agacaggaac     900
   acccccttcc cactctgccc catgtctcaa gttgtagtgg cccttcctcc agatctctgc      960
   caccatctta gaaaggaaca ctgaaagaag aaactgaaat tataagctga cagcataaag    1020
   aggatgagta aaacctaaaa tcattgttca aatgaatgaa tcaagagaag tttaaaccac    1080
   tttggactaa aatgtgtgaa tcctttttcc tgctatccag cagatgagaa gctggtaaca    1140
   gagaccaaaa tagtttggag actaaagaat cattgcacat ttcactgctg agttgtattg 1200
   tgagtaattt tagttgacct cacttttgta aatcttgcac acgggcatcc atatctgcac 1260
   agagatatgt taacagtggt aaatgctgca tgaggagatt gggtgatttt tactttcgtt 1320
   tttgtgctct tctttcttat tgttcttact tatttacgat taccctatcg ttttccaaaa 1380
   tgtaaaaggc cattttgaaa gcctaattca aacctcttca ctattttgta tctaagtatt 1440
   caccttgatt gagactgggt agacaggtga aaaccatatc aggtttttaa ttttttaatt 1500
   tttaattatt tatttattta tttatttttt gagatggagt ctggctgtcg cccaggctgg 1560
   agtgcagcgg cgtgatcaca gttcactgca gcctcaacct tctaggctca agggattctc 1620
   ccacctcagc cccccaagta gttgggacca cacgtatgcg ccaccatgcc tggctaattt 1680
   cttatttttt tgtagagata ggatctcact atattgtcca ggctggtctt gaattcctgg 1740
   gctcaggtga gcctcccacc tgggcctccc aaagtactgg gattacaggc atgagccaag 1800
   gtcccctgcc catatgagat tttctgtctc tgatcccatg cagctagtaa tcaaggactt 1860
   ggctgctgac tctggaggac ctgcatgctt tcttgagctg tgaacttcag tgctaaaagc 1920
   tcataggcag ccctgaaacc caaaccaaaa ggttctatgg tttatcatcc cgatcatgtt 1980
   gattttatag aaataacaca tgaattaaag acactaccct caaactgagc aaaacttaag 2040
   taattttttt aaagtttgac ctgtttttaa atcactcttg gagaaaaagg aaaataaata 2100
   caaataatta acggtgaata caggctacta tacctttgtt ctccagaatt agcagttctg 2160
   ttcttttctt gctttagatg ctgaagtgca gaaggacact ctgtgattgt acgtgtgtaa 2220
   ctgacaaaat gtgtattttt tttctcagct gctatggatt ggattatgct attatgaata 2280
   agaatgctga tgggagcaca cacaaaccat ttgttcctca gtccattttc ctcctcaaaa 2340
   gcctggaatg tgccattgat cagtgggaga tgtacctgga cagacccatg aaaagagatc 2400
   aacaagttcc acccaaggga ccctattttt cctaatttca tttgaaatgg cttctaattg 2460
   tccttctttc attcctgctt cctaccagtt ttacagcttt ttctggtttc aaatgtgaac 2520
   tcacatacac tctcattttt cctcatcaca accccaagtg acccaatggt cctcactttc 2580
   gatataagta aaggaggctc tgcattaagg gcttgtccaa ggcacgcagc tgagaggcgc 2640
   taggactggc tccatttcca tctctattct cactgacttt gactacccag aaccccaaca 2700
   tgtggggcct cagtattcga tcaattattc tattaagaag caaaaacaat tccccgcatt 2760
   ggccccagtt attaagcatt tctcagattt accttgagaa atgcccatcg gcctgtatat 2820
   tcacatcttc acccttgtcc cttcctccta gaaaggagaa agtcagttgg atgccctctg 2880
   aggaactagt gcatggctta actgtccttc catgactcct gccttatctg ttttctattt 2940
   tcctcctttt ccaccgaagt ctataatctc aagaaaagca ggcactggcc ttagggctcc 3000
   tggcctaaga aatatcaagt ccagtgagaa atcccattga ctgacccctc ctgcttaccc 3060
   ctttgtgatg gagaagctcc caggggtttg ctttttgcat gttaccaggc ctaactcagc 3120
   atcaccaggg gcaagaaaag gaaagtaacc taaactaatg ctgcttataa ttgtaattat   3180
   tgtaatagtt aattactgtg attgtacatg tgtaacagac aaaatgtgta tttttttcac   3240
   agctgctgtg gattggatta tgccatttgg aataagaatg ctgttaagag cacacaagcc   3300
   aggttcctca agtccgtagc aaatttttca aaagttaaat ttaaaaatca ctacatttga   3360
   atctagtgac aggagaaatg gacatggata gagactaaag atctagccca aattttatat   3420
   ttacttgtta gaggattttg aacaaattac taaatttctt caaggttcaa tttccccatt   3480
   aactataatg aatggctcat cattatgggg ccctggagaa gcataattac ttgtaattgt   3540
   aataatcatt gttattatta ttatacatat tttgctttta aatggataag gatttttaag   3600
   gtatatgtaa actgtaaaac ataaaatgca aaatgccgta agagacagta gtaataataa   3660
   tgattattat attgttatca ttatctagcc tgttttttcc tgttttgtat ttcttccttt   3720
   aaatgctttc agaaatctgt atccccattc ttcaccacca ccccacaaca tttctgcttc   3780
   ttttcccatg ccgggtcatg ctaactttga aagcttcagc tctttccttc ctcaatcctt   3840
   ttcctggcac ctctgatatg ccttttgaaa ttcatgttaa agaatcccta ggctgctatc   3900
   acatgtggca tctttgttga gtacatgaat aaatcaactg gtgtgtttta cgaaggatga   3960
   ttatgcttca ttgtgggatt gtatttttct tcttctatca cagggagaag tgaa atg      4017
                                                               Met
                                                               1
   aca acc tca cta gat aca gtt gag acc ttt ggt acc aca tcc tac tat      4065
   Thr Thr Ser Leu Asp Thr Val Glu Thr Phe Gly Thr Thr Ser Tyr Tyr
               5                   10                  15
   gat gac gtg ggc ctg ctc tgt gaa aaa gct gat acc aga gca ctg atg      4113
   Asp Asp Val Gly Leu Leu Cys Glu Lys Ala Asp Thr Arg Ala Leu Met
           20                  25                  30
   gcc cag ttt gtg ccc ccg ctg tac tcc ctg gtg ttc act gtg ggc ctc      4161
   Ala Gln Phe Val Pro Pro Leu Tyr Ser Leu Val Phe Thr Val Gly Leu
       35                  40                  45
   ttg ggc aat gtg gtg gtg gtg atg atc ctc ata aaa tac agg agg ctc      4209
   Leu Gly Asn Val Val Val Val Met Ile Leu Ile Lys Tyr Arg Arg Leu
   50                  55                  60                  65
   cga att atg acc aac atc tac ctg ctc aac ctg gcc att tcg gac ctg      4257
   Arg Ile Met Thr Asn Ile Tyr Leu Leu Asn Leu Ala Ile Ser Asp Leu
                   70                  75                  80
   ctc ttc ctc gtc acc ctt cca ttc tgg atc cac tat gtc agg ggg cat      4305
   Leu Phe Leu Val Thr Leu Pro Phe Trp Ile His Tyr Val Arg Gly His
               85                  90                  95
   aac tgg gtt ttt ggc cat ggc atg tgt aag ctc ctc tca ggg ttt tat      4353
   Asn Trp Val Phe Gly His Gly Met Cys Lys Leu Leu Ser Gly Phe Tyr
           100                 105                 110
   cac aca ggc ttg tac agc gag atc ttt ttc ata atc ctg ctg aca atc      4401
   His Thr Gly Leu Tyr Ser Glu Ile Phe Phe Ile Ile Leu Leu Thr Ile
       115                 120                 125
   gac agg tac ctg gcc att gtc cat gct gtg ttt gcc ctt cga gcc cgg      4449
   Asp Arg Tyr Leu Ala Ile Val His Ala Val Phe Ala Leu Arg Ala Arg
   130                 135                 140                 145
   act gtc act ttt ggt gtc atc acc agc atc gtc acc tgg ggc ctg gca      4497
   Thr Val Thr Phe Gly Val Ile Thr Ser Ile Val Thr Trp Gly Leu Ala
                   150                 155                 160
   gtg cta gca gct ctt cct gaa ttt atc ttc tat gag act gaa gag ttg      4545
   Val Leu Ala Ala Leu Pro Glu Phe Ile Phe Tyr Glu Thr Glu Glu Leu
               165                 170                 175
   ttt gaa gag act ctt tgc agt gct ctt tac cca gag gat aca gta tat      4593
   Phe Glu Glu Thr Leu Cys Ser Ala Leu Tyr Pro Glu Asp Thr Val Tyr
           180                 185                 190
   agc tgg agg cat ttc cac act ctg aga atg acc atc ttc tgt ctc gtt      4641
   Ser Trp Arg His Phe His Thr Leu Arg Met Thr Ile Phe Cys Leu Val
       195                 200                 205
   ctc cct ctg ctc gtt atg gcc atc tgc tac aca gga atc atc aaa acg      4689
   Leu Pro Leu Leu Val Met Ala Ile Cys Tyr Thr Gly Ile Ile Lys Thr
   210                 215                 220                 225
   ctg ctg agg tgc ccc agt aaa aaa aag tac aag gcc atc cgg ctc att      4737
   Leu Leu Arg Cys Pro Ser Lys Lys Lys Tyr Lys Ala Ile Arg Leu Ile
                   230                 235                 240
   ttt gtc atc atg gcg gtg ttt ttc att ttc tgg aca ccc tac aat gtg      4785
   Phe Val Ile Met Ala Val Phe Phe Ile Phe Trp Thr Pro Tyr Asn Val
               245                 250                 255
   gct atc ctt ctc tct tcc tat caa tcc atc tta ttt gga aat gac tgt      4833
   Ala Ile Leu Leu Ser Ser Tyr Gln Ser Ile Leu Phe Gly Asn Asp Cys
           260                 265                 270
   gag cgg agc aag cat ctg gac ctg gtc atg ctg gtg aca gag gtg atc      4881
   Glu Arg Ser Lys His Leu Asp Leu Val Met Leu Val Thr Glu Val Ile
       275                 280                 285
   gcc tac tcc cac tgc tgc atg aac ccg gtg atc tac gcc ttt gtt gga      4929
   Ala Tyr Ser His Cys Cys Met Asn Pro Val Ile Tyr Ala Phe Val Gly
   290                 295                 300                 305
   gag agg ttc cgg aag tac ctg cgc cac ttc ttc cac agg cac ttg ctc      4977
   Glu Arg Phe Arg Lys Tyr Leu Arg His Phe Phe His Arg His Leu Leu
                   310                 315                 320
   atg cac ctg ggc aga tac atc cca ttc ctt cct agt gag aag ctg gaa      5025
   Met His Leu Gly Arg Tyr Ile Pro Phe Leu Pro Ser Glu Lys Leu Glu
               325                 330                 335
   aga acc agc tct gtc tct cca tcc aca gca gag ccg gaa ctc tct att      5073
   Arg Thr Ser Ser Val Ser Pro Ser Thr Ala Glu Pro Glu Leu Ser Ile
           340                 345                 350
   gtg ttt tag gtcagatgca gaaaattgcc taaagaggaa ggaccaagga              5122
   Val Phe
       355
   gatgaagcaa acacattaag ccttccacac tcacctct                            5160
<210> 6
   <211> 355
   <212> PRT
   <213> Homo sapiens
<400> 6
   Met Thr Thr Ser Leu Asp Thr Val Glu Thr Phe Gly Thr Thr Ser Tyr
   1               5                   10                  15
   Tyr Asp Asp Val Gly Leu Leu Cys Glu Lys Ala Asp Thr Arg Ala Leu
               20                  25                  30
   Met Ala Gln Phe Val Pro Pro Leu Tyr Ser Leu Val Phe Thr Val Gly
           35                  40                  45
   Leu Leu Gly Asn Val Val Val Val Met Ile Leu Ile Lys Tyr Arg Arg
       50                  55                  60
   Leu Arg Ile Met Thr Asn Ile Tyr Leu Leu Asn Leu Ala Ile Ser Asp
   65                  70                  75                  80
   Leu Leu Phe Leu Val Thr Leu Pro Phe Trp Ile His Tyr Val Arg Gly
                   85                  90                  95
   His Asn Trp Val Phe Gly His Gly Met Cys Lys Leu Leu Ser Gly Phe
               100                 105                 110
   Tyr His Thr Gly Leu Tyr Ser Glu Ile Phe Phe Ile Ile Leu Leu Thr
           115                 120                 125
   Ile Asp Arg Tyr Leu Ala Ile Val His Ala Val Phe Ala Leu Arg Ala
       130                 135                 140
   Arg Thr Val Thr Phe Gly Val Ile Thr Ser Ile Val Thr Trp Gly Leu
   145                 150                 155                 160
   Ala Val Leu Ala Ala Leu Pro Glu Phe Ile Phe Tyr Glu Thr Glu Glu
                   165                 170                 175
   Leu Phe Glu Glu Thr Leu Cys Ser Ala Leu Tyr Pro Glu Asp Thr Val
               180                 185                 190
   Tyr Ser Trp Arg His Phe His Thr Leu Arg Met Thr Ile Phe Cys Leu
           195                 200                 205
   Val Leu Pro Leu Leu Val Met Ala Ile Cys Tyr Thr Gly Ile Ile Lys
       210                 215                 220
   Thr Leu Leu Arg Cys Pro Ser Lys Lys Lys Tyr Lys Ala Ile Arg Leu
   225                 230                 235                 240
   Ile Phe Val Ile Met Ala Val Phe Phe Ile Phe Trp Thr Pro Tyr Asn
                   245                 250                 255
   Val Ala Ile Leu Leu Ser Ser Tyr Gln Ser Ile Leu Phe Gly Asn Asp
               260                 265                 270
   Cys Glu Arg Ser Lys His Leu Asp Leu Val Met Leu Val Thr Glu Val
           275                 280                 285
   Ile Ala Tyr Ser His Cys Cys Met Asn Pro Val Ile Tyr Ala Phe Val
       290                 295                 300
   Gly Glu Arg Phe Arg Lys Tyr Leu Arg His Phe Phe His Arg His Leu
   305                 310                 315                 320
   Leu Met His Leu Gly Arg Tyr Ile Pro Phe Leu Pro Ser Glu Lys Leu
                   325                 330                 335
   Glu Arg Thr Ser Ser Val Ser Pro Ser Thr Ala Glu Pro Glu Leu Ser
               340                 345                 350
   Ile Val Phe
           355
<210> 7
   <211> 1680
   <212> DNA
   <213> Homo sapiens
<220>
   <221> CDS
   <222> (361)..(1485)
<400> 7
   agaaacagga gcagatgtac agggtttgcc tgactcacac tcaaggttgc ataagcaaga      60
   tttcaaaatt aatcctattc tggagacctc aacccaatgt acaatgttcc tgactggaaa     120
   agaagaacta tatttttctg attttttttt tcaaatcttt accattagtt gccctgtatc     180
   tccgccttca ctttctgcag gaaactttat ttcctacttc tgcatgccaa gtttctacct     240
   ctagatctgt ttggttcagt tgctgagaag cctgacatac caggactgcc tgagacaagc     300
   cacaagctga acagagaaag tggattgaac aaggacgcat ttccccagta catccacaac     360
   atg ctg tcc aca tct cgt tct cgg ttt atc aga aat acc aac gag agc       408
   Met Leu Ser Thr Ser Arg Ser Arg Phe Ile Arg Asn Thr Asn Glu Ser
   1               5                   10                  15
   ggt gaa gaa gtc acc acc ttt ttt gat tat gat tac ggt gct ccc tgt       456
   Gly Glu Glu Val Thr Thr Phe Phe Asp Tyr Asp Tyr Gly Ala Pro Cys
               20                  25                  30
   cat aaa ttt gac gtg aag caa att ggg gcc caa ctc ctg cct ccg ctc       504
   His Lys Phe Asp Val Lys Gln Ile Gly Ala Gln Leu Leu Pro Pro Leu
               35                  40                  45
   tac tcg ctg gtg ttc atc ttt ggt ttt gtg ggc aac atg ctg gtc gtc       552
   Tyr Ser Leu Val Phe Ile Phe Gly Phe Val Gly Asn Met Leu Val Val
       50                  55                  60
   ctc atc tta ata aac tgc aaa aag ctg aag tgc ttg act gac att tac       600
   Leu Ile Leu Ile Asn Cys Lys Lys Leu Lys Cys Leu Thr Asp Ile Tyr
   65                  70                  75                  80
   ctg ctc aac ctg gcc atc tct gat ctg ctt ttt ctt att act ctc cca       648
   Leu Leu Asn Leu Ala Ile Ser Asp Leu Leu Phe Leu Ile Thr Leu Pro
                   85                  90                  95
   ttg tgg gct cac tct gct gca aat gag tgg gtc ttt ggg aat gca atg       696
   Leu Trp Ala His Ser Ala Ala Asn Glu Trp Val Phe Gly Asn Ala Met
               100                 105                 110
   tgc aaa tta ttc aca ggg ctg tat cac atc ggt tat ttt ggc gga atc       744
   Cys Lys Leu Phe Thr Gly Leu Tyr His Ile Gly Tyr Phe Gly Gly Ile
           115                 120                 125
   ttc ttc atc atc ctc ctg aca atc gat aga tac ctg gct att gtc cat       792
   Phe Phe Ile Ile Leu Leu Thr Ile Asp Arg Tyr Leu Ala Ile Val His
       130                 135                 140
   gct gtg ttt gct tta aaa gcc agg acg gtc acc ttt ggg gtg gtg aca       840
   Ala Val Phe Ala Leu Lys Ala Arg Thr Val Thr Phe Gly Val Val Thr
   145                 150                 155                 160
   agt gtg atc acc tgg ttg gtg gct gtg ttt gct tct gtc cca gga atc       888
   Ser Val Ile Thr Trp Leu Val Ala Val Phe Ala Ser Val Pro Gly Ile
                   165                 170                 175
   atc ttt act aaa tgc cag aaa gaa gat tct gtt tat gtc tgt ggc cct       936
   Ile Phe Thr Lys Cys Gln Lys Glu Asp Ser Val Tyr Val Cys Gly Pro
               180                 185                 190
   tat ttt cca cga gga tgg aat aat ttc cac aca ata atg agg aac att       984
   Tyr Phe Pro Arg Gly Trp Asn Asn Phe His Thr Ile Met Arg Asn Ile
           195                 200                 205
   ttg ggg ctg gtc ctg ccg ctg ctc atc atg gtc atc tgc tac tcg gga      1032
   Leu Gly Leu Val Leu Pro Leu Leu Ile Met Val Ile Cys Tyr Ser Gly
       210                 215                 220
   atc ctg aaa acc ctg ctt cgg tgt cga aac gag aag aag agg cat agg      1080
   Ile Leu Lys Thr Leu Leu Arg Cys Arg Asn Glu Lys Lys Arg His Arg
   225                 230                 235                 240
   gca gtg aga gtc atc ttc acc atc atg att gtt tac ttt ctc ttc tgg      1128
   Ala Val Arg Val Ile Phe Thr Ile Met Ile Val Tyr Phe Leu Phe Trp
                   245                 250                 255
   act ccc tat aat att gtc att ctc ctg aac acc ttc cag gaa ttc ttc      1176
   Thr Pro Tyr Asn Ile Val Ile Leu Leu Asn Thr Phe Gln Glu Phe Phe
               260                 265                 270
   ggc ctg agt aac tgt gaa agc acc agt caa ctg gac caa gcc acg cag      1224
   Gly Leu Ser Asn Cys Glu Ser Thr Ser Gln Leu Asp Gln Ala Thr Gln
           275                 280                 285
   gtg aca gag act ctt ggg atg act cac tgc tgc atc aat ccc atc atc      1272
   Val Thr Glu Thr Leu Gly Met Thr His Cys Cys Ile Asn Pro Ile Ile
       290                 295                 300
   tat gcc ttc gtt ggg gag aag ttc aga agc ctt ttt cac ata gct ctt      1320
   Tyr Ala Phe Val Gly Glu Lys Phe Arg Ser Leu Phe His Ile Ala Leu
   305                 310                 315                 320
   ggc tgt agg att gcc cca ctc caa aaa cca gtg tgt gga ggt cca gga      1368
   Gly Cys Arg Ile Ala Pro Leu Gln Lys Pro Val Cys Gly Gly Pro Gly
                   325                 330                 335
   gtg aga cca gga aag aat gtg aaa gtg act aca caa gga ctc ctc gat      1416
   Val Arg Pro Gly Lys Asn Val Lys Val Thr Thr Gln Gly Leu Leu Asp
               340                 345                 350
   ggt cgt gga aaa gga aag tca att ggc aga gcc cct gaa gcc agt ctt      1464
   Gly Arg Gly Lys Gly Lys Ser Ile Gly Arg Ala Pro Glu Ala Ser Leu
           355                 360                 365
   cag gac aaa gaa gga gcc tag agacagaaat gacagatctc tgctttggaa         1515
   Gln Asp Lys Glu Gly Ala
       370
   atcacacgtc tggcttcaca gatgtgtgat tcacagtgtg aatcttggtg tctacgttac    1575
   caggcaggaa ggctgagagg agagagactc cagctgggtt ggaaaacagt attttccaaa    1635
   ctaccttcca gttcctcatt tttgaataca ggcatagagt tcaga                    1680
<210> 8
   <211> 374
   <212> PRT
   <213> Homo sapiens
<400> 8
   Met Leu Ser Thr Ser Arg Ser Arg Phe Ile Arg Asn Thr Asn Glu Ser
   1 5 10 15
   Gly Glu Glu Val Thr Thr Phe Phe Asp Tyr Asp Tyr Gly Ala Pro Cys
   20 25 30
   His Lys Phe Asp Val Lys Gln Ile Gly Ala Gln Leu Leu Pro Pro Leu
   35 40 45
   Tyr Ser Leu Val Phe Ile Phe Gly Phe Val Gly Asn Met Leu Val Val
   50 55 60
   Leu Ile Leu Ile Asn Cys Lys Lys Leu Lys Cys Leu Thr Asp Ile Tyr
   65 70 75 80
   Leu Leu Asn Leu Ala Ile Ser Asp Leu Leu Phe Leu Ile Thr Leu Pro
   85 90 95
   Leu Trp Ala His Ser Ala Ala Asn Glu Trp Val Phe Gly Asn Ala Met
   100 105 110
   Cys Lys Leu Phe Thr Gly Leu Tyr His Ile Gly Tyr Phe Gly Gly Ile
   115 120 125
   Phe Phe Ile Ile Leu Leu Thr Ile Asp Arg Tyr Leu Ala Ile Val His
       130                 135                 140
   Ala Val Phe Ala Leu Lys Ala Arg Thr Val Thr Phe Gly Val Val Thr
   145                 150                 155                 160
   Ser Val Ile Thr Trp Leu Val Ala Val Phe Ala Ser Val Pro Gly Ile
                   165                 170                 175
   Ile Phe Thr Lys Cys Gln Lys Glu Asp Ser Val Tyr Val Cys Gly Pro
               180                 185                 190
   Tyr Phe Pro Arg Gly Trp Asn Asn Phe His Thr Ile Met Arg Asn Ile
           195                 200                 205
   Leu Gly Leu Val Leu Pro Leu Leu Ile Met Val Ile Cys Tyr Ser Gly
       210                 215                 220
   Ile Leu Lys Thr Leu Leu Arg Cys Arg Asn Glu Lys Lys Arg His Arg
   225                 230                 235                 240
   Ala Val Arg Val Ile Phe Thr Ile Met Ile Val Tyr Phe Leu Phe Trp
                   245                 250                 255
   Thr Pro Tyr Asn Ile Val Ile Leu Leu Asn Thr Phe Gln Glu Phe Phe
               260                 265                 270
   Gly Leu Ser Asn Cys Glu Ser Thr Ser Gln Leu Asp Gln Ala Thr Gln
           275                 280                 285
   Val Thr Glu Thr Leu Gly Met Thr His Cys Cys Ile Asn Pro Ile Ile
       290                 295                 300
   Tyr Ala Phe Val Gly Glu Lys Phe Arg Ser Leu Phe His Ile Ala Leu
   305                 310                 315                 320
   Gly Cys Arg Ile Ala Pro Leu Gln Lys Pro Val Cys Gly Gly Pro Gly
                   325                 330                 335
   Val Arg Pro Gly Lys Asn Val Lys Val Thr Thr Gln Gly Leu Leu Asp
               340                 345                 350
   Gly Arg Gly Lys Gly Lys Ser Ile Gly Arg Ala Pro Glu Ala Ser Leu
           355                 360                 365
   Gln Asp Lys Glu Gly Ala
       370
<210> 9
   <211> 1260
   <212> DNA
   <213> Homo sapiens
<220>
   <221> CDS
   <222> (72)..(1139)
<400> 9
   ataaaaaccc agaaagcccc agaaacaaag acttcacgga caaagtccct tggaaccaga      60
   gagaagccgg g atg gaa act cca aac acc aca gag gac tat gac acg acc      110
                Met Glu Thr Pro Asn Thr Thr Glu Asp Tyr Asp Thr Thr
                1               5                   10
   aca gag ttt gac tat ggg gat gca act ccg tgc cag aag gtg aac gag      158
   Thr Glu Phe Asp Tyr Gly Asp Ala Thr Pro Cys Gln Lys Val Asn Glu
       15                  20                  25
   agg gcc ttt ggg gcc caa ctg ctg ccc cct ctg tac tcc ttg gta ttt      206
   Arg Ala Phe Gly Ala Gln Leu Leu Pro Pro Leu Tyr Ser Leu Val Phe
   30                  35                  40                  45
   gtc att ggc ctg gtt gga aac atc ctg gtg gtc ctg gtc ctt gtg caa      254
   Val Ile Gly Leu Val Gly Asn Ile Leu Val Val Leu Val Leu Val Gln
                   50                  55                  60
   tac aag agg cta aaa aac atg acc agc atc tac ctc ctg aac ctg gcc      302
   Tyr Lys Arg Leu Lys Asn Met Thr Ser Ile Tyr Leu Leu Asn Leu Ala
               65                  70                  75
   att tct gac ctg ctc ttc ctg ttc acg ctt ccc ttc tgg atc gac tac      350
   Ile Ser Asp Leu Leu Phe Leu Phe Thr Leu Pro Phe Trp Ile Asp Tyr
           80                  85                  90
   aag ttg aag gat gac tgg gtt ttt ggt gat gcc atg tgt aag atc ctc      398
   Lys Leu Lys Asp Asp Trp Val Phe Gly Asp Ala Met Cys Lys Ile Leu
       95                  100                 105
   tct ggg ttt tat tac aca ggc ttg tac agc gag atc ttt ttc atc atc      446
   Ser Gly Phe Tyr Tyr Thr Gly Leu Tyr Ser Glu Ile Phe Phe Ile Ile
   110                 115                 120                 125
   ctg ctg acg att gac agg tac ctg gcc atc gtc cac gcc gtg ttt gcc      494
   Leu Leu Thr Ile Asp Arg Tyr Leu Ala Ile Val His Ala Val Phe Ala
                   130                 135                 140
   ttg cgg gca cgg acc gtc act ttt ggt gtc atc acc agc atc atc att      542
   Leu Arg Ala Arg Thr Val Thr Phe Gly Val Ile Thr Ser Ile Ile Ile
               145                 150                 155
   tgg gcc ctg gcc atc ttg gct tcc atg cca ggc tta tac ttt tcc aag      590
   Trp Ala Leu Ala Ile Leu Ala Ser Met Pro Gly Leu Tyr Phe Ser Lys
           160                 165                 170
   acc caa tgg gaa ttc act cac cac acc tgc agc ctt cac ttt cct cac      638
   Thr Gln Trp Glu Phe Thr His His Thr Cys Ser Leu His Phe Pro His
       175                 180                 185
   gaa agc cta cga gag tgg aag ctg ttt cag gct ctg aaa ctg aac ctc      686
   Glu Ser Leu Arg Glu Trp Lys Leu Phe Gln Ala Leu Lys Leu Asn Leu
   190                 195                 200                 205
   ttt ggg ctg gta ttg cct ttg ttg gtc atg atc atc tgc tac aca ggg      734
   Phe Gly Leu Val Leu Pro Leu Leu Val Met Ile Ile Cys Tyr Thr Gly
                   210                 215                 220
   att ata aag att ctg cta aga cga cca aat gag aag aaa tcc aaa gct      782
   Ile Ile Lys Ile Leu Leu Arg Arg Pro Asn Glu Lys Lys Ser Lys Ala
               225                 230                 235
   gtc cgt ttg att ttt gtc atc atg atc atc ttt ttt ctc ttt tgg acc      830
   Val Arg Leu Ile Phe Val Ile Met Ile Ile Phe Phe Leu Phe Trp Thr
           240                 245                 250
   ccc tac aat ttg act ata ctt att tct gtt ttc caa gac ttc ctg ttc      878
   Pro Tyr Asn Leu Thr Ile Leu Ile Ser Val Phe Gln Asp Phe Leu Phe
       255                 260                 265
   acc cat gag tgt gag cag agc aga cat ttg gac ctg gct gtg caa gtg      926
   Thr His Glu Cys Glu Gln Ser Arg His Leu Asp Leu Ala Val Gln Val
   270                 275                 280                 285
   acg gag gtg atc gcc tac acg cac tgc tgt gtc aac cca gtg atc tac      974
   Thr Glu Val Ile Ala Tyr Thr His Cys Cys Val Asn Pro Val Ile Tyr
                   290                 295                 300
   gcc ttc gtt ggt gag agg ttc cgg aag tac ctg cgg cag ttg ttc cac     1022
   Ala Phe Val Gly Glu Arg Phe Arg Lys Tyr Leu Arg Gln Leu Phe His
               305                 310                 315
   agg cgt gtg gct gtg cac ctg gtt aaa tgg ctc ccc ttc ctc tcc gtg     1070
   Arg Arg Val Ala Val His Leu Val Lys Trp Leu Pro Phe Leu Ser Val
           320                 325                 330
   gac agg ctg gag agg gtc agc tcc aca tct ccc tcc aca ggg gag cat     1118
   Asp Arg Leu Glu Arg Val Ser Ser Thr Ser Pro Ser Thr Gly Glu His
       335                 340                 345
   gaa ctc tct gct ggg ttc tga ctcagaccat aggaggccaa cccaaaataa        1169
   Glu Leu Ser Ala Gly Phe
   350                 355
   gcaggcgtga cctgccaggc acactgagcc agcagcctgg ctctcccagc caggttctga   1229
   ctcttggcac agcatggagt cacagccact t                                  1260
<210> 10
   <211> 355
   <212> PRT
   <213> Homo sapiens
<400> 10
   Met Glu Thr Pro Asn Thr Thr Glu Asp Tyr Asp Thr Thr Thr Glu Phe
   1               5                   10                  15
   Asp Tyr Gly Asp Ala Thr Pro Cys Gln Lys Val Asn Glu Arg Ala Phe
               20                  25                  30
   Gly Ala Gln Leu Leu Pro Pro Leu Tyr Ser Leu Val Phe Val Ile Gly
           35                  40                  45
   Leu Val Gly Asn Ile Leu Val Val Leu Val Leu Val Gln Tyr Lys Arg
   50 55 60
   Leu Lys Asn Met Thr Ser Ile Tyr Leu Leu Asn Leu Ala Ile Ser Asp
   65 70 75 80
   Leu Leu Phe Leu Phe Thr Leu Pro Phe Trp Ile Asp Tyr Lys Leu Lys
   85 90 95
   Asp Asp Trp Val Phe Gly Asp Ala Met Cys Lys Ile Leu Ser Gly Phe
   100 105 110
   Tyr Tyr Thr Gly Leu Tyr Ser Glu Ile Phe Phe Ile Ile Leu Leu Thr
   115 120 125
   Ile Asp Arg Tyr Leu Ala Ile Val His Ala Val Phe Ala Leu Arg Ala
   130 135 140
   Arg Thr Val Thr Phe Gly Val Ile Thr Ser Ile Ile Ile Trp Ala Leu
   145 150 155 160
   Ala Ile Leu Ala Ser Met Pro Gly Leu Tyr Phe Ser Lys Thr Gln Trp
   165 170 175
   Glu Phe Thr His His Thr Cys Ser Leu His Phe Pro His Glu Ser Leu
   180 185 190
   Arg Glu Trp Lys Leu Phe Gln Ala Leu Lys Leu Asn Leu Phe Gly Leu
   195 200 205
   Val Leu Pro Leu Leu Val Met Ile Ile Cys Tyr Thr Gly Ile Ile Lys
   210 215 220
   Ile Leu Leu Arg Arg Pro Asn Glu Lys Lys Ser Lys Ala Val Arg Leu
   225 230 235 240
   Ile Phe Val Ile Met Ile Ile Phe Phe Leu Phe Trp Thr Pro Tyr Asn
   245 250 255
   Leu Thr Ile Leu Ile Ser Val Phe Gln Asp Phe Leu Phe Thr His Glu
   260 265 270
   Cys Glu Gln Ser Arg His Leu Asp Leu Ala Val Gln Val Thr Glu Val
   275 280 285
   Ile Ala Tyr Thr His Cys Cys Val Asn Pro Val Ile Tyr Ala Phe Val
   290 295 300
   Gly Glu Arg Phe Arg Lys Tyr Leu Arg Gln Leu Phe His Arg Arg Val
   305 310 315 320
   Ala Val His Leu Val Lys Trp Leu Pro Phe Leu Ser Val Asp Arg Leu
   325 330 335
   Glu Arg Val Ser Ser Thr Ser Pro Ser Thr Gly Glu His Glu Leu Ser
   340 345 350
   Ala Gly Phe
   355
<210> 11
   <211> 1380
   <212> DNA
   <213> Homo sapiens
<220>
   <221> CDS
   <222> (183)..(1265)
<400> 11
   cgggggtttt gatcttcttc cccttctttt cttccccttc ttctttcctt cctccctccc     60
   tctctcattt cccttctcct tctccctcag tctccacatt caacattgac aagtccattc    120
   agaaaagcaa gctgcttctg gttgggccca gacctgcctt gaggagcctg tagagttaaa    180
   aa atg aac ccc acg gat ata gca gat acc acc ctc gat gaa agc ata       227
      Met Asn Pro Thr Asp Ile Ala Asp Thr Thr Leu Asp Glu Ser Ile
      1               5                   10                  15
   tac agc aat tac tat ctg tat gaa agt atc ccc aag cct tgc acc aaa      275
   Tyr Ser Asn Tyr Tyr Leu Tyr Glu Ser Ile Pro Lys Pro Cys Thr Lys
                   20                  25                  30
   gaa ggc atc aag gca ttt ggg gag ctc ttc ctg ccc cca ctg tat tcc      323
   Glu Gly Ile Lys Ala Phe Gly Glu Leu Phe Leu Pro Pro Leu Tyr Ser
               35                  40                  45
   ttg gtt ttt gta ttt ggt ctg ctt gga aat tct gtg gtg gtt ctg gtc      371
   Leu Val Phe Val Phe Gly Leu Leu Gly Asn Ser Val Val Val Leu Val
           50                  55                  60
   ctg ttc aaa tac aag cgg ctc agg tcc atg act gat gtg tac ctg ctc      419
   Leu Phe Lys Tyr Lys Arg Leu Arg Ser Met Thr Asp Val Tyr Leu Leu
       65                  70                  75
   aac ctt gcc atc tcg gat ctg ctc ttc gtg ttt tcc ctc cct ttt tgg      467
   Asn Leu Ala Ile Ser Asp Leu Leu Phe Val Phe Ser Leu Pro Phe Trp
   80                  85                  90                  95
   ggc tac tat gca gca gac cag tgg gtt ttt ggg cta ggt ctg tgc aag      515
   Gly Tyr Tyr Ala Ala Asp Gln Trp Val Phe Gly Leu Gly Leu Cys Lys
                   100                 105                 110
   atg att tcc tgg atg tac ttg gtg ggc ttt tac agt ggc ata ttc ttt      563
   Met Ile Ser Trp Met Tyr Leu Val Gly Phe Tyr Ser Gly Ile Phe Phe
               115                 120                 125
   gtc atg ctc atg agc att gat aga tac ctg gcg ata gtg cac gcg gtg      611
   Val Met Leu Met Ser Ile Asp Arg Tyr Leu Ala Ile Val His Ala Val
           130                 135                 140
   ttt tcc ttg agg gca agg acc ttg act tat ggg gtc atc acc agt ttg       659
   Phe Ser Leu Arg Ala Arg Thr Leu Thr Tyr Gly Val Ile Thr Ser Leu
       145                 150                 155
   gct aca tgg tca gtg gct gtg ttc gcc tcc ctt cct ggc ttt ctg ttc       707
   Ala Thr Trp Ser Val Ala Val Phe Ala Ser Leu Pro Gly Phe Leu Phe
   160                 165                 170                 175
   agc act tgt tat act gag cgc aac cat acc tac tgc aaa acc aag tac       755
   Ser Thr Cys Tyr Thr Glu Arg Asn His Thr Tyr Cys Lys Thr Lys Tyr
                   180                 185                 190
   tct ctc aac tcc acg acg tgg aag gtt ctc agc tcc ctg gaa atc aac       803
   Ser Leu Asn Ser Thr Thr Trp Lys Val Leu Ser Ser Leu Glu Ile Asn
               195                 200                 205
   att ctc gga ttg gtg atc ccc tta ggg atc atg ctg ttt tgc tac tcc       851
   Ile Leu Gly Leu Val Ile Pro Leu Gly Ile Met Leu Phe Cys Tyr Ser
           210                 215                 220
   atg atc atc agg acc ttg cag cat tgt aaa aat gag aag aag aac aag       899
   Met Ile Ile Arg Thr Leu Gln His Cys Lys Asn Glu Lys Lys Asn Lys
       225                 230                 235
   gcg gtg aag atg atc ttt gcc gtg gtg gtc ctc ttc ctt ggg ttc tgg       947
   Ala Val Lys Met Ile Phe Ala Val Val Val Leu Phe Leu Gly Phe Trp
   240                 245                 250                 255
   aca cct tac aac ata gtg ctc ttc cta gag acc ctg gtg gag cta gaa       995
   Thr Pro Tyr Asn Ile Val Leu Phe Leu Glu Thr Leu Val Glu Leu Glu
                   260                 265                 270
   gtc ctt cag gac tgc acc ttt gaa aga tac ttg gac tat gcc atc cag      1043
   Val Leu Gln Asp Cys Thr Phe Glu Arg Tyr Leu Asp Tyr Ala Ile Gln
               275                 280                 285
   gcc aca gaa act ctg gct ttt gtt cac tgc tgc ctt aat ccc atc atc      1091
   Ala Thr Glu Thr Leu Ala Phe Val His Cys Cys Leu Asn Pro Ile Ile
           290                 295                 300
   tac ttt ttt ctg ggg gag aaa ttt cgc aag tac atc cta cag ctc ttc      1139
   Tyr Phe Phe Leu Gly Glu Lys Phe Arg Lys Tyr Ile Leu Gln Leu Phe
       305                 310                 315
   aaa acc tgc agg ggc ctt ttt gtg ctc tgc caa tac tgt ggg ctc ctc      1187
   Lys Thr Cys Arg Gly Leu Phe Val Leu Cys Gln Tyr Cys Gly Leu Leu
   320                 325                 330                 335
   caa att tac tct gct gac acc ccc agc tca tct tac acg cag tcc acc      1235
   Gln Ile Tyr Ser Ala Asp Thr Pro Ser Ser Ser Tyr Thr Gln Ser Thr
                   340                 345                 350
   atg gat cat gat ctt cat gat gct ctg tag gaaaaatgaa atggtgaaat        1285
   Met Asp His Asp Leu His Asp Ala Leu
               355                 360
   gcagagtcaa tgaacttttc cacattcaga gcttacttta aaattggtat ttttaggtaa    1345
   gagatccctg agccagtgtc aggaggaagg cttac                               1380
<210> 12
   <211> 360
   <212> PRT
   <213> Homo sapiens
<400> 12
   Met Asn Pro Thr Asp Ile Ala Asp Thr Thr Leu Asp Glu Ser Ile Tyr
   1               5                   10                  15
   Ser Asn Tyr Tyr Leu Tyr Glu Ser Ile Pro Lys Pro Cys Thr Lys Glu
               20                  25                  30
   Gly Ile Lys Ala Phe Gly Glu Leu Phe Leu Pro Pro Leu Tyr Ser Leu
           35                  40                  45
   Val Phe Val Phe Gly Leu Leu Gly Asn Ser Val Val Val Leu Val Leu
       50                  55                  60
   Phe Lys Tyr Lys Arg Leu Arg Ser Met Thr Asp Val Tyr Leu Leu Asn
   65                  70                  75                  80
   Leu Ala Ile Ser Asp Leu Leu Phe Val Phe Ser Leu Pro Phe Trp Gly
                   85                  90                  95
   Tyr Tyr Ala Ala Asp Gln Trp Val Phe Gly Leu Gly Leu Cys Lys Met
               100                 105                 110
   Ile Ser Trp Met Tyr Leu Val Gly Phe Tyr Ser Gly Ile Phe Phe Val
           115                 120                 125
   Met Leu Met Ser Ile Asp Arg Tyr Leu Ala Ile Val His Ala Val Phe
       130                 135                 140
   Ser Leu Arg Ala Arg Thr Leu Thr Tyr Gly Val Ile Thr Ser Leu Ala
   145                 150                 155                 160
   Thr Trp Ser Val Ala Val Phe Ala Ser Leu Pro Gly Phe Leu Phe Ser
                   165                 170                 175
   Thr Cys Tyr Thr Glu Arg Asn His Thr Tyr Cys Lys Thr Lys Tyr Ser
               180                 185                 190
   Leu Asn Ser Thr Thr Trp Lys Val Leu Ser Ser Leu Glu Ile Asn Ile
           195                 200                 205
   Leu Gly Leu Val Ile Pro Leu Gly Ile Met Leu Phe Cys Tyr Ser Met
       210                 215                 220
   Ile Ile Arg Thr Leu Gln His Cys Lys Asn Glu Lys Lys Asn Lys Ala
   225                 230                 235                 240
   Val Lys Met Ile Phe Ala Val Val Val Leu Phe Leu Gly Phe Trp Thr
                   245                 250                 255
   Pro Tyr Asn Ile Val Leu Phe Leu Glu Thr Leu Val Glu Leu Glu Val
               260                 265                 270
   Leu Gln Asp Cys Thr Phe Glu Arg Tyr Leu Asp Tyr Ala Ile Gln Ala
           275                 280                 285
   Thr Glu Thr Leu Ala Phe Val His Cys Cys Leu Asn Pro Ile Ile Tyr
       290                 295                 300
   Phe Phe Leu Gly Glu Lys Phe Arg Lys Tyr Ile Leu Gln Leu Phe Lys
   305                 310                 315                 320
   Thr Cys Arg Gly Leu Phe Val Leu Cys Gln Tyr Cys Gly Leu Leu Gln
                   325                 330                 335
   Ile Tyr Ser Ala Asp Thr Pro Ser Ser Ser Tyr Thr Gln Ser Thr Met
               340                 345                 350
   Asp His Asp Leu His Asp Ala Leu
           355                 360
<210> 13
   <211> 1320
   <212> DNA
   <213> Homo spaiens
<220>
   <221> CDS
   <222> (139)..(1206)
<400> 13
   tttgtagtgg gaggatacct ccagagaggc tgctgctcat tgagctgcac tcacatgagg      60
   atacagactt tgtgaagaag gaattggcaa cactgaaacc tccagaacaa aggctgtcac     120
   taaggtcccg ctgccttg atg gat tat aca ctt gac ctc agt gtg aca aca       171
                       Met Asp Tyr Thr Leu Asp Leu Ser Val Thr Thr
                       1               5                   10
   gtg acc gac tac tac tac cct gat atc ttc tca agc ccc tgt gat gcg       219
   Val Thr Asp Tyr Tyr Tyr Pro Asp Ile Phe Ser Ser Pro Cys Asp Ala
               15                  20                  25
   gaa ctt att cag aca aat ggc aag ttg ctc ctt gct gtc ttt tat tgc       267
   Glu Leu Ile Gln Thr Asn Gly Lys Leu Leu Leu Ala Val Phe Tyr Cys
           30                  35                  40
   ctc ctg ttt gta ttc agt ctt ctg gga aac agc ctg gtc atc ctg gtc       315
   Leu Leu Phe Val Phe Ser Leu Leu Gly Asn Ser Leu Val Ile Leu Val
       45                  50                  55
   ctt gtg gtc tgc aag aag ctg agg agc atc aca gat gta tac ctc ttg       363
   Leu Val Val Cys Lys Lys Leu Arg Ser Ile Thr Asp Val Tyr Leu Leu
   60                  65                  70                  75
   aac ctg gcc ctg tct gac ctg ctt ttt gtc ttc tcc ttc ccc ttt cag       411
   Asn Leu Ala Leu Ser Asp Leu Leu Phe Val Phe Ser Phe Pro Phe Gln
                   80                  85                  90
   acc tac tat ctg ctg gac cag tgg gtg ttt ggg act gta atg tgc aaa       459
   Thr Tyr Tyr Leu Leu Asp Gln Trp Val Phe Gly Thr Val Met Cys Lys
               95                  100                 105
   gtg gtg tct ggc ttt tat tac att ggc ttc tac agc agc atg ttt ttc       507
   Val Val Ser Gly Phe Tyr Tyr Ile Gly Phe Tyr Ser Ser Met Phe Phe
           110                 115                 120
   atc acc ctc atg agt gtg gac agg tac ctg gct gtt gtc cat gcc gtg       555
   Ile Thr Leu Met Ser Val Asp Arg Tyr Leu Ala Val Val His Ala Val
       125                 130                 135
   tat gcc cta aag gtg agg acg atc agg atg ggc aca acg ctg tgc ctg       603
   Tyr Ala Leu Lys Val Arg Thr Ile Arg Met Gly Thr Thr Leu Cys Leu
   140                 145                 150                 155
   gca gta tgg cta acc gcc att atg gct acc atc cca ttg cta gtg ttt       651
   Ala Val Trp Leu Thr Ala Ile Met Ala Thr Ile Pro Leu Leu Val Phe
                   160                 165                 170
   tac caa gtg gcc tct gaa gat ggt gtt cta cag tgt tat tca ttt tac       699
   Tyr Gln Val Ala Ser Glu Asp Gly Val Leu Gln Cys Tyr Ser Phe Tyr
               175                 180                 185
   aat caa cag act ttg aag tgg aag atc ttc acc aac ttc aaa atg aac       747
   Asn Gln Gln Thr Leu Lys Trp Lys Ile Phe Thr Asn Phe Lys Met Asn
           190                 195                 200
   att tta ggc ttg ttg atc cca ttc acc atc ttt atg ttc tgc tac att       795
   Ile Leu Gly Leu Leu Ile Pro Phe Thr Ile Phe Met Phe Cys Tyr Ile
       205                 210                 215
   aaa atc ctg cac cag ctg aag agg tgt caa aac cac aac aag acc aag       843
   Lys Ile Leu His Gln Leu Lys Arg Cys Gln Asn His Asn Lys Thr Lys
   220                 225                 230                 235
   gcc atc agg ttg gtg ctc att gtg gtc att gca tct tta ctt ttc tgg       891
   Ala Ile Arg Leu Val Leu Ile Val Val Ile Ala Ser Leu Leu Phe Trp
                   240                 245                 250
   gtc cca ttc aac gtg gtt ctt ttc ctc act tcc ttg cac agt atg cac       939
   Val Pro Phe Asn Val Val Leu Phe Leu Thr Ser Leu His Ser Met His
               255                 260                 265
   atc ttg gat gga tgt agc ata agc caa cag ctg act tat gcc acc cat       987
   Ile Leu Asp Gly Cys Ser Ile Ser Gln Gln Leu Thr Tyr Ala Thr His
           270                 275                 280
   gtc aca gaa atc att tcc ttt act cac tgc tgt gtg aac cct gtt atc      1035
   Val Thr Glu Ile Ile Ser Phe Thr His Cys Cys Val Asn Pro Val Ile
       285                 290                 295
   tat gct ttt gtt ggg gag aag ttc aag aaa cac ctc tca gaa ata ttt      1083
   Tyr Ala Phe Val Gly Glu Lys Phe Lys Lys His Leu Ser Glu Ile Phe
   300                 305                 310                 315
   cag aaa agt tgc agc caa atc ttc aac tac cta gga aga caa atg cct      1131
   Gln Lys Ser Cys Ser Gln Ile Phe Asn Tyr Leu Gly Arg Gln Met Pro
                   320                 325                 330
   agg gag agc tgt gaa aag tca tca tcc tgc cag cag cac tcc tcc cgt      1179
   Arg Glu Ser Cys Glu Lys Ser Ser Ser Cys Gln Gln His Ser Ser Arg
               335                 340                 345
   tcc tcc agc gta gac tac att ttg tga ggatcaatga agactaaata            1226
   Ser Ser Ser Val Asp Tyr Ile Leu
           350                 355
   taaaaaacat tttcttgaat ggcatgctag tagcagtgag caaaggtgtg ggtgtgaaag    1286
   gtttccaaaa aaagttcagc atgaaggatg ccat                                1320
<210> 14
   <211> 355
   <212> PRT
   <213> Homo spaiens
<400> 14
   Met Asp Tyr Thr Leu Asp Leu Ser Val Thr Thr Val Thr Asp Tyr Tyr
   1 5 10 15
   Tyr Pro Asp Ile Phe Ser Ser Pro Cys Asp Ala Glu Leu Ile Gln Thr
   20 25 30
   Asn Gly Lys Leu Leu Leu Ala Val Phe Tyr Cys Leu Leu Phe Val Phe
   35 40 45
   Ser Leu Leu Gly Asn Ser Leu Val Ile Leu Val Leu Val Val Cys Lys
   50 55 60
   Lys Leu Arg Ser Ile Thr Asp Val Tyr Leu Leu Asn Leu Ala Leu Ser
   65 70 75 80
   Asp Leu Leu Phe Val Phe Ser Phe Pro Phe Gln Thr Tyr Tyr Leu Leu
   85 90 95
   Asp Gln Trp Val Phe Gly Thr Val Met Cys Lys Val Val Ser Gly Phe
   100 105 110
   Tyr Tyr Ile Gly Phe Tyr Ser Ser Met Phe Phe Ile Thr Leu Met Ser
   115 120 125
   Val Asp Arg Tyr Leu Ala Val Val His Ala Val Tyr Ala Leu Lys Val
   130 135 140
   Arg Thr Ile Arg Met Gly Thr Thr Leu Cys Leu Ala Val Trp Leu Thr
   145 150 155 160
   Ala Ile Met Ala Thr Ile Pro Leu Leu Val Phe Tyr Gln Val Ala Ser
   165 170 175
   Glu Asp Gly Val Leu Gln Cys Tyr Ser Phe Tyr Asn Gln Gln Thr Leu
               180                 185                 190
   Lys Trp Lys Ile Phe Thr Asn Phe Lys Met Asn Ile Leu Gly Leu Leu
           195                 200                 205
   Ile Pro Phe Thr Ile Phe Met Phe Cys Tyr Ile Lys Ile Leu His Gln
       210                 215                 220
   Leu Lys Arg Cys Gln Asn His Asn Lys Thr Lys Ala Ile Arg Leu Val
   225                 230                 235                 240
   Leu Ile Val Val Ile Ala Ser Leu Leu Phe Trp Val Pro Phe Asn Val
                   245                 250                 255
   Val Leu Phe Leu Thr Ser Leu His Ser Met His Ile Leu Asp Gly Cys
               260                 265                 270
   Ser Ile Ser Gln Gln Leu Thr Tyr Ala Thr His Val Thr Glu Ile Ile
           275                 280                 285
   Ser Phe Thr His Cys Cys Val Asn Pro Val Ile Tyr Ala Phe Val Gly
       290                 295                 300
   Glu Lys Phe Lys Lys His Leu Ser Glu Ile Phe Gln Lys Ser Cys Ser
   305                 310                 315                 320
   Gln Ile Phe Asn Tyr Leu Gly Arg Gln Met Pro Arg Glu Ser Cys Glu
                   325                 330                 335
   Lys Ser Ser Ser Cys Gln Gln His Ser Ser Arg Ser Ser Ser Val Asp
               340                 345                 350
   Tyr Ile Leu
           355
<210> 15
   <211> 1186
   <212> DNA
   <213> Homo sapiens
<220>
   <221> CDS
   <222> (60)..(1169)
<400> 15
   tgtcccaggg agagttgcat cgccctccac agagcaggct tgcatctgac tgacccacc       59
   atg aca ccc aca gac ttc aca agc cct att cct aac atg gct gat gac       107
   Met Thr Pro Thr Asp Phe Thr Ser Pro Ile Pro Asn Met Ala Asp Asp
   1               5                   10                  15
   tat ggc tct gaa tcc aca tct tcc atg gaa gac tac gtt aac ttc aac      155
   Tyr Gly Ser Glu Ser Thr Ser Ser Met Glu Asp Tyr Val Asn Phe Asn
               20                  25                  30
   ttc act gac ttc tac tgt gag aaa aac aat gtc agg cag ttt gcg agc      203
   Phe Thr Asp Phe Tyr Cys Glu Lys Asn Asn Val Arg Gln Phe Ala Ser
           35                  40                  45
   cat ttc ctc cca ccc ttg tac tgg ctc gtg ttc atc gtg ggt gcc ttg      251
   His Phe Leu Pro Pro Leu Tyr Trp Leu Val Phe Ile Val Gly Ala Leu
       50                  55                  60
   ggc aac agt ctt gtt atc ctt gtc tac tgg tac tgc aca aga gtg aag      299
   Gly Asn Ser Leu Val Ile Leu Val Tyr Trp Tyr Cys Thr Arg Val Lys
   65                  70                  75                  80
   acc atg acc gac atg ttc ctt ttg aat ttg gca att gct gac ctc ctc      347
   Thr Met Thr Asp Met Phe Leu Leu Asn Leu Ala Ile Ala Asp Leu Leu
                   85                  90                  95
   ttt ctt gtc act ctt ccc ttc tgg gcc att gct gct gct gac cag tgg      395
   Phe Leu Val Thr Leu Pro Phe Trp Ala Ile Ala Ala Ala Asp Gln Trp
               100                 105                 110
   aag ttc cag acc ttc atg tgc aag gtg gtc aac agc atg tac aag atg      443
   Lys Phe Gln Thr Phe Met Cys Lys Val Val Asn Ser Met Tyr Lys Met
           115                 120                 125
   aac ttc tac agc tgt gtg ttg ctg atc atg tgc atc agc gtg gac agg      491
   Asn Phe Tyr Ser Cys Val Leu Leu Ile Met Cys Ile Ser Val Asp Arg
       130                 135                 140
   tac att gcc att gcc cag gcc atg aga gca cat act tgg agg gag aaa      539
   Tyr Ile Ala Ile Ala Gln Ala Met Arg Ala His Thr Trp Arg Glu Lys
   145                 150                 155                 160
   agg ctt ttg tac agc aaa atg gtt tgc ttt acc atc tgg gta ttg gca      587
   Arg Leu Leu Tyr Ser Lys Met Val Cys Phe Thr Ile Trp Val Leu Ala
                   165                 170                 175
   gct gct ctc tgc atc cca gaa atc tta tac agc caa atc aag gag gaa      635
   Ala Ala Leu Cys Ile Pro Glu Ile Leu Tyr Ser Gln Ile Lys Glu Glu
               180                 185                 190
   tcc ggc att gct atc tgc acc atg gtt tac cct agc gat gag agc acc      683
   Ser Gly Ile Ala Ile Cys Thr Met Val Tyr Pro Ser Asp Glu Ser Thr
           195                 200                 205
   aaa ctg aag tca gct gtc ttg acc ctg aag gtc att ctg ggg ttc ttc      731
   Lys Leu Lys Ser Ala Val Leu Thr Leu Lys Val Ile Leu Gly Phe Phe
       210                 215                 220
   ctt ccc ttc gtg gtc atg gct tgc tgc tat acc atc atc att cac acc      779
   Leu Pro Phe Val Val Met Ala Cys Cys Tyr Thr Ile Ile Ile His Thr
   225                 230                 235                 240
   ctg ata caa gcc aag aag tct tcc aag cac aaa gcc cta aaa gtg acc      827
   Leu Ile Gln Ala Lys Lys Ser Ser Lys His Lys Ala Leu Lys Val Thr
                   245                 250                 255
   atc act gtc ctg acc gtc ttt gtc ttg tct cag ttt ccc tac aac tgc      875
   Ile Thr Val Leu Thr Val Phe Val Leu Ser Gln Phe Pro Tyr Asn Cys
               260                 265                 270
   att ttg ttg gtg cag acc att gac gcc tat gcc atg ttc atc tcc aac      923
   Ile Leu Leu Val Gln Thr Ile Asp Ala Tyr Ala Met Phe Ile Ser Asn
           275                 280                 285
   tgt gcc gtt tcc acc aac att gac atc tgc ttc cag gtc acc cag acc      971
   Cys Ala Val Ser Thr Asn Ile Asp Ile Cys Phe Gln Val Thr Gln Thr
       290                 295                 300
   atc gcc ttc ttc cac agt tgc ctg aac cct gtt ctc tat gtt ttt gtg     1019
   Ile Ala Phe Phe His Ser Cys Leu Asn Pro Val Leu Tyr Val Phe Val
   305                 310                 315                 320
   ggt gag aga ttc cgc cgg gat ctc gtg aaa acc ctg aag aac ttg ggt     1067
   Gly Glu Arg Phe Arg Arg Asp Leu Val Lys Thr Leu Lys Asn Leu Gly
                   325                 330                 335
   tgc atc agc cag gcc cag tgg gtt tca ttt aca agg aga gag gga agc     1115
   Cys Ile Ser Gln Ala Gln Trp Val Ser Phe Thr Arg Arg Glu Gly Ser
               340                 345                 350
   ttg aag ctg tcg tct atg ttg ctg gag aca acc tca gga gca ctc tcc     1163
   Leu Lys Leu Ser Ser Met Leu Leu Glu Thr Thr Ser Gly Ala Leu Ser
           355                 360                 365
   ctc tga ggggtcttct ctgaggt                                          1186
   Leu
<210> 16
   <211> 369
   <212> PRT
   <213> Homo sapiens
<400> 16
   Met Thr Pro Thr Asp Phe Thr Ser Pro Ile Pro Asn Met Ala Asp Asp
   1               5                   10                  15
   Tyr Gly Ser Glu Ser Thr Ser Ser Met Glu Asp Tyr Val Asn Phe Asn
               20                  25                  30
   Phe Thr Asp Phe Tyr Cys Glu Lys Asn Asn Val Arg Gln Phe Ala Ser
           35                  40                  45
   His Phe Leu Pro Pro Leu Tyr Trp Leu Val Phe Ile Val Gly Ala Leu
       50                  55                  60
   Gly Asn Ser Leu Val Ile Leu Val Tyr Trp Tyr Cys Thr Arg Val Lys
   65                  70                  75                  80
   Thr Met Thr Asp Met Phe Leu Leu Asn Leu Ala Ile Ala Asp Leu Leu
                   85                  90                  95
   Phe Leu Val Thr Leu Pro Phe Trp Ala Ile Ala Ala Ala Asp Gln Trp
               100                 105                 110
   Lys Phe Gln Thr Phe Met Cys Lys Val Val Asn Ser Met Tyr Lys Met
   115 120 125
   Asn Phe Tyr Ser Cys Val Leu Leu Ile Met Cys Ile Ser Val Asp Arg
   130 135 140
   Tyr Ile Ala Ile Ala Gln Ala Met Arg Ala His Thr Trp Arg Glu Lys
   145 150 155 160
   Arg Leu Leu Tyr Ser Lys Met Val Cys Phe Thr Ile Trp Val Leu Ala
   165 170 175
   Ala Ala Leu Cys Ile Pro Glu Ile Leu Tyr Ser Gln Ile Lys Glu Glu
   180 185 190
   Ser Gly Ile Ala Ile Cys Thr Met Val Tyr Pro Ser Asp Glu Ser Thr
   195 200 205
   Lys Leu Lys Ser Ala Val Leu Thr Leu Lys Val Ile Leu Gly Phe Phe
   210 215 220
   Leu Pro Phe Val Val Met Ala Cys Cys Tyr Thr Ile Ile Ile His Thr
   225 230 235 240
   Leu Ile Gln Ala Lys Lys Ser Ser Lys His Lys Ala Leu Lys Val Thr
   245 250 255
   Ile Thr Val Leu Thr Val Phe Val Leu Ser Gln Phe Pro Tyr Asn Cys
   260 265 270
   Ile Leu Leu Val Gln Thr Ile Asp Ala Tyr Ala Met Phe Ile Ser Asn
   275 280 285
   Cys Ala Val Ser Thr Asn Ile Asp Ile Cys Phe Gln Val Thr Gln Thr
   290 295 300
   Ile Ala Phe Phe His Ser Cys Leu Asn Pro Val Leu Tyr Val Phe Val
   305 310 315 320
   Gly Glu Arg Phe Arg Arg Asp Leu Val Lys Thr Leu Lys Asn Leu Gly
   325 330 335
   Cys Ile Ser Gln Ala Gln Trp Val Ser Phe Thr Arg Arg Glu Gly Ser
   340 345 350
   Leu Lys Leu Ser Ser Met Leu Leu Glu Thr Thr Ser Gly Ala Leu Ser
   355 360 365
   Leu
<210> 17
   <211> 2400
   <212> DNA
   <213> Homo sapiens
<220>
   <221> CDS
   <222> (1178)..(2260)
<400> 17
caccctccac acccaggctt ccccagagca ggatctgtgc tgtccctgtg gcaagggcag    60
   agccccagga gctagactaa atctgagagg aacaggggag tcaaggctag atggaggcac   120
   acatgcagga caggaagggt ctccagcagg gctgaaagca ccaagcaagg gtagtgcaaa   180
   ccctgtcttc tctgcttcct gctgcccctc tcttattggg tagcagactg gctgtgtctg   240
   cttatcccgc atggtggcac atggctgccc agagctcccc aaataacctg ttacgtttcc   300
   acccacaggg agaatcaggc agcccgcttg cttattgttt ttaatatact tttccaaact   360
   acacagacat tcccaaagcg ggtctccttc tacccaaaag agaaacgctg ggccttacta   420
   attaactgaa gactctgcta gctcgagcct tccaaaactc catgccacaa ttgttcaaac   480
   cattttccag aatacatctt ttaaaataac ttttagaaaa ttgaacaaag ggattcatgt   540
   ctcagcttta tagtcaaacc atgatctttt ctgagggtat agcccatttg gagttcctgc   600
   ttaatcccct gattaaaaac tgaatggggc tgagtgcagt gctcatgcct atattcccag   660
   cactttggga gacagaggca ggagaattac ttaaggtcag gagtttaaga ccatcctgag   720
   caacataaca agtccccatc tctaagacaa aaaaaaaaga actgagtgac atctcacatc   780
   tcacatttct gaacattaaa cccagccttg atagccaaag atgctcgcca ctgaaggatc   840
   caggtagtat tgagggttct gtggggatta tccaaagaga actttctaca aagttttagg   900
   tgatggcgat gctaaaagaa atgctaagaa tttctctctt atattaaaga gaactatggt   960
   cctctcataa aatgtaccat ttatcaccaa atttatctca taacctaaga gctaccactt  1020
   acaaatttga agggaaaaat tactacattg taatactcaa gccaacacaa agaatcctat  1080
   cccagtttct tgagtggatg ggcaagaata tggggaattt attatgcagt aaccttcatc  1140
   tctcttctat aggtcaggat ttaagtttac ctcaaaa atg gaa gat ttt aac atg  1195
   gag agt gac agc ttt gaa gat ttc tgg aaa ggt gaa gat ctt agt aat     1243
   tac agt tac agc tct acc ctg ccc cct ttt cta cta gat gcc gcc cca     1291
   tgt gaa cca gaa tcc ctg gaa atc aac aag tat ttt gtg gtc att atc     1339
   tat gcc ctg gta ttc ctg ctg agc ctg ctg gga aac tcc ctc gtg atg 1387
   ctg gtc atc tta tac agc agg gtc ggc cgc tcc gtc act gat gtc tac 1435
   ctg ctg aac cta gcc ttg gcc gac cta ctc ttt gcc ctg acc ttg ccc 1483
   atc tgg gcc gcc tcc aag gtg aat ggc tgg att ttt ggc aca ttc ctg 1531
   tgc aag gtg gtc tca ctc ctg aag gaa gtc aac ttc tat agt ggc atc 1579
   ctg cta ctg gcc tgc atc agt gtg gac cgt tac ctg gcc att gtc cat 1627
   gcc aca cgc aca ctg acc cag aag cgc tac ttg gtc aaa ttc ata tgt 1675
   ctc agc atc tgg ggt ctg tcc ttg ctc ctg gcc ctg cct gtc tta ctt 1723
   ttc cga agg acc gtc tac tca tcc aat gtt agc cca gcc tgc tat gag 1771
   gac atg ggc aac aat aca gca aac tgg cgg atg ctg tta cgg atc ctg 1819
   ccc cag tcc ttt ggc ttc atc gtg cca ctg ctg atc atg ctg ttc tgc 1867
   tac gga ttc acc ctg cgt acg ctg ttt aag gcc cac atg ggg cag aag 1915
   cac cgg gcc atg cgg gtc atc ttt gct gtc gtc ctc atc ttc ctg ctc 1963
   tgc tgg ctg ccc tac aac ctg gtc ctg ctg gca gac acc ctc atg agg 2011
   acc cag gtg atc cag gag acc tgt gag cgc cgc aat cac atc gac cgg 2059
   gct ctg gat gcc acc gag att ctg ggc atc ctt cac agc tgc ctc aac 2107
   ccc ctc atc tac gcc ttc att ggc cag aag ttt cgc cat gga ctc ctc      2155
   aag att cta gct ata cat ggc ttg atc agc aag gac tcc ctg ccc aaa      2203
   gac agc agg cct tcc ttt gtt ggc tct tct tca ggg cac act tcc act      2251
   act ctc taa gacctcctgc ctaagtgcag cccgtggggt tcctcccttc              2300
   tcttcacagt cacattccaa gcctcatgtc cactggttct tcttggtctc agtgtcaatg    2360
   cagcccccat tgtggtcaca ggaagtagag gaggccacgt                          2400
<210> 18
   <211> 360
   <212> PRT
   <213> Homo sapiens
<400> 18
<210> 19
   <211> 1029
   <212> DNA
   <213> Homo sapiens
<220>
   <221> CDS
   <222> (1)..(1029)
<400> 19
   atg gca gag cat gat tac cat gaa gac tat ggg ttc agc agt ttc aat      48
   Met Ala Glu His Asp Tyr His Glu Asp Tyr Gly Phe Ser Ser Phe Asn
   1               5                   10                  15
   gac agc agc cag gag gag cat caa gac ttc ctg cag ttc agc aag gtc      96
   Asp Ser Ser Gln Glu Glu His Gln Asp Phe Leu Gln Phe Ser Lys Val
               20                  25                  30
   ttt ctg ccc tgc atg tac ctg gtg gtg ttt gtc tgt ggt ctg gtg ggg     144
   Phe Leu Pro Cys Met Tyr Leu Val Val Phe Val Cys Gly Leu Val Gly
           35                  40                  45
   aac tct ctg gtg ctg gtc ata tcc atc ttc tac cat aag ttg cag agc     192
   Asn Ser Leu Val Leu Val Ile Ser Ile Phe Tyr His Lys Leu Gln Ser
       50                  55                  60
   ctg acg gat gtg ttc ctg gtg aac cta ccc ctg gct gac ctg gtg ttt     240
   Leu Thr Asp Val Phe Leu Val Asn Leu Pro Leu Ala Asp Leu Val Phe
   65                  70                  75                  80
   gtc tgc act ctg ccc ttc tgg gcc tat gca ggc atc cat gaa tgg gtg     288
   Val Cys Thr Leu Pro Phe Trp Ala Tyr Ala Gly Ile His Glu Trp Val
                   85                  90                  95
   ttt ggc cag gtc atg tgc aag agc cta ctg ggc atc tac act att aac     336
   Phe Gly Gln Val Met Cys Lys Ser Leu Leu Gly Ile Tyr Thr Ile Asn
               100                 105                 110
   ttc tac acg tcc atg ctc atc ctc acc tgc atc act gtg gat cgt ttc     384
   Phe Tyr Thr Ser Met Leu Ile Leu Thr Cys Ile Thr Val Asp Arg Phe
           115                 120                 125
   att gta gtg gtt aag gcc acc aag gcc tac aac cag caa gcc aag agg     432
   Ile Val Val Val Lys Ala Thr Lys Ala Tyr Asn Gln Gln Ala Lys Arg
       130                 135                 140
   atg acc tgg ggc aag gtc acc agc ttg ctc atc tgg gtg ata tcc ctg     480
   Met Thr Trp Gly Lys Val Thr Ser Leu Leu Ile Trp Val Ile Ser Leu
   145                 150                 155                 160
   ctg gtt tcc ttg ccc caa att atc tat ggc aat gtc ttt aat ctc gac     528
   Leu Val Ser Leu Pro Gln Ile Ile Tyr Gly Asn Val Phe Asn Leu Asp
                   165                 170                 175
   aag ctc ata tgt ggt tac cat gac gag gca att tcc act gtg gtt ctt     576
   Lys Leu Ile Cys Gly Tyr His Asp Glu Ala Ile Ser Thr Val Val Leu
               180                 185                 190
   gcc acc cag atg aca ctg ggg ttc ttc ttg cca ctg ctc acc atg att     624
   Ala Thr Gln Met Thr Leu Gly Phe Phe Leu Pro Leu Leu Thr Met Ile
           195                 200                 205
   gtc tgc tat tca gtc ata atc aaa aca ctg ctt cat gct gga ggc ttc     672
   Val Cys Tyr Ser Val Ile Ile Lys Thr Leu Leu His Ala Gly Gly Phe
       210                 215                 220
   cag aag cac aga tct cta aag atc atc ttc ctg gtg atg gct gtg ttc     720
   Gln Lys His Arg Ser Leu Lys Ile Ile Phe Leu Val Met Ala Val Phe
   225                 230                 235                 240
   ctg ctg acc cag atg ccc ttc aac ctc atg aag ttc atc cgc agc aca     768
   Leu Leu Thr Gln Met Pro Phe Asn Leu Met Lys Phe Ile Arg Ser Thr
                   245                 250                 255
   cac tgg gaa tac tat gcc atg acc agc ttt cac tac acc atc atg gtg       816
   His Trp Glu Tyr Tyr Ala Met Thr Ser Phe His Tyr Thr Ile Met Val
               260                 265                 270
   aca gag gcc atc gca tac ctg agg gcc tgc ctt aac cct gtg ctc tat       864
   Thr Glu Ala Ile Ala Tyr Leu Arg Ala Cys Leu Asn Pro Val Leu Tyr
           275                 280                 285
   gcc ttt gtc agc ctg aag ttt cga aag aac ttc tgg aaa ctt gtg aag       912
   Ala Phe Val Ser Leu Lys Phe Arg Lys Asn Phe Trp Lys Leu Val Lys
       290                 295                 300
   gac att ggt tgc ctc cct tac ctt ggg gtc tca cat caa tgg aaa tct       960
   Asp Ile Gly Cys Leu Pro Tyr Leu Gly Val Ser His Gln Trp Lys Ser
   305                 310                 315                 320
   tct gag gac aat tcc aag act ttt tct gcc tcc cac aat gtg gag gcc      1008
   Ser Glu Asp Asn Ser Lys Thr Phe Ser Ala Ser His Asn Val Glu Ala
                   325                 330                 335
   acc agc atg ttc cag tta tag                                          1029
   Thr Ser Met Phe Gln Leu
               340
<210> 20
   <211> 342
   <212> PRT
   <213> Homo sapiens
<400> 20
   Met Ala Glu His Asp Tyr His Glu Asp Tyr Gly Phe Ser Ser Phe Asn
   1               5                   10                  15
   Asp Ser Ser Gln Glu Glu His Gln Asp Phe Leu Gln Phe Ser Lys Val
               20                  25                  30
   Phe Leu Pro Cys Met Tyr Leu Val Val Phe Val Cys Gly Leu Val Gly
           35                  40                  45
   Asn Ser Leu Val Leu Val Ile Ser Ile Phe Tyr His Lys Leu Gln Ser
       50                  55                  60
   Leu Thr Asp Val Phe Leu Val Asn Leu Pro Leu Ala Asp Leu Val Phe
   65                  70                  75                  80
   Val Cys Thr Leu Pro Phe Trp Ala Tyr Ala Gly Ile His Glu Trp Val
                   85                  90                  95
   Phe Gly Gln Val Met Cys Lys Ser Leu Leu Gly Ile Tyr Thr Ile Asn
               100                 105                 110
   Phe Tyr Thr Ser Met Leu Ile Leu Thr Cys Ile Thr Val Asp Arg Phe
           115                 120                 125
   Ile Val Val Val Lys Ala Thr Lys Ala Tyr Asn Gln Gln Ala Lys Arg
       130                 135                 140
   Met Thr Trp Gly Lys Val Thr Ser Leu Leu Ile Trp Val Ile Ser Leu
   145                 150                 155                 160
   Leu Val Ser Leu Pro Gln Ile Ile Tyr Gly Asn Val Phe Asn Leu Asp
                   165                 170                 175
   Lys Leu Ile Cys Gly Tyr His Asp Glu Ala Ile Ser Thr Val Val Leu
               180                 185                 190
   Ala Thr Gln Met Thr Leu Gly Phe Phe Leu Pro Leu Leu Thr Met Ile
           195                 200                 205
   Val Cys Tyr Ser Val Ile Ile Lys Thr Leu Leu His Ala Gly Gly Phe
       210                 215                 220
   Gln Lys His Arg Ser Leu Lys Ile Ile Phe Leu Val Met Ala Val Phe
   225                 230                 235                 240
   Leu Leu Thr Gln Met Pro Phe Asn Leu Met Lys Phe Ile Arg Ser Thr
                   245                 250                 255
   His Trp Glu Tyr Tyr Ala Met Thr Ser Phe His Tyr Thr Ile Met Val
               260                 265                 270
   Thr Glu Ala Ile Ala Tyr Leu Arg Ala Cys Leu Asn Pro Val Leu Tyr
           275                 280                 285
   Ala Phe Val Ser Leu Lys Phe Arg Lys Asn Phe Trp Lys Leu Val Lys
       290                 295                 300
   Asp Ile Gly Cys Leu Pro Tyr Leu Gly Val Ser His Gln Trp Lys Ser
   305                 310                 315                 320
   Ser Glu Asp Asn Ser Lys Thr Phe Ser Ala Ser His Asn Val Glu Ala
                   325                 330                 335
   Thr Ser Met Phe Gln Leu
               340
<210> 21
   <211> 1260
   <212> DNA
   <213> Homo sapiens
<220>
   <221> CDS
   <222> (93)..(1160)
<400> 21
   gaaatactcg tctctggtaa agtctgagca ggacagggtg gctgactggc agatccagag      60
   gttcccttgg cagtccacgc caggccttca cc atg gat cag ttc cct gaa tca      113
                                        Met Asp Gln Phe Pro Glu Ser
                                        1               5
   gtg aca gaa aac ttt gag tac gat gat ttg gct gag gcc tgt tat att      161
   Val Thr Glu Asn Phe Glu Tyr Asp Asp Leu Ala Glu Ala Cys Tyr Ile
           10                  15                  20
   ggg gac atc gtg gtc ttt ggg act gtg ttc ctg tcc ata ttc tac tcc      209
   Gly Asp Ile Val Val Phe Gly Thr Val Phe Leu Ser Ile Phe Tyr Ser
       25                  30                  35
   gtc atc ttt gcc att ggc ctg gtg gga aat ttg ttg gta gtg ttt gcc      257
   Val Ile Phe Ala Ile Gly Leu Val Gly Asn Leu Leu Val Val Phe Ala
   40                  45                  50                  55
   ctc acc aac agc aag aag ccc aag agt gtc acc gac att tac ctc ctg      305
   Leu Thr Asn Ser Lys Lys Pro Lys Ser Val Thr Asp Ile Tyr Leu Leu
                   60                  65                  70
   aac ctg gcc ttg tct gat ctg ctg ttt gta gcc act ttg ccc ttc tgg      353
   Asn Leu Ala Leu Ser Asp Leu Leu Phe Val Ala Thr Leu Pro Phe Trp
               75                  80                  85
   act cac tat ttg ata aat gaa aag ggc ctc cac aat gcc atg tgc aaa      401
   Thr His Tyr Leu Ile Asn Glu Lys Gly Leu His Asn Ala Met Cys Lys
           90                  95                  100
   ttc act acc gcc ttc ttc ttc atc ggc ttt ttt gga agc ata ttc ttc      449
   Phe Thr Thr Ala Phe Phe Phe Ile Gly Phe Phe Gly Ser Ile Phe Phe
       105                 110                 115
   atc acc gtc atc agc att gat agg tac ctg gcc atc gtc ctg gcc gcc      497
   Ile Thr Val Ile Ser Ile Asp Arg Tyr Leu Ala Ile Val Leu Ala Ala
   120                 125                 130                 135
   aac tcc atg aac aac cgg acc gtg cag cat ggc gtc acc atc agc cta      545
   Asn Ser Met Asn Asn Arg Thr Val Gln His Gly Val Thr Ile Ser Leu
                   140                 145                 150
   ggc gtc tgg gca gca gcc att ttg gtg gca gca ccc cag ttc atg ttc      593
   Gly Val Trp Ala Ala Ala Ile Leu Val Ala Ala Pro Gln Phe Met Phe
               155                 160                 165
   aca aag cag aaa gaa aat gaa tgc ctt ggt gac tac ccc gag gtc ctc      641
   Thr Lys Gln Lys Glu Asn Glu Cys Leu Gly Asp Tyr Pro Glu Val Leu
           170                 175                 180
   cag gaa atc tgg ccc gtg ctc cgc aat gtg gaa aca aat ttt ctt ggc      689
   Gln Glu Ile Trp Pro Val Leu Arg Asn Val Glu Thr Asn Phe Leu Gly
       185                 190                 195
   ttc cta ctc ccc ctg ctc att atg agt tat tgc tac ttc aga atc atc      737
   Phe Leu Leu Pro Leu Leu Ile Met Ser Tyr Cys Tyr Phe Arg Ile Ile
   200                 205                 210                 215
   cag acg ctg ttt tcc tgc aag aac cac aag aaa gcc aaa gcc att aaa      785
   Gln Thr Leu Phe Ser Cys Lys Asn His Lys Lys Ala Lys Ala Ile Lys
                   220                 225                 230
   ctg atc ctt ctg gtg gtc atc gtg ttt ttc ctc ttc tgg aca ccc tac      833
   Leu Ile Leu Leu Val Val Ile Val Phe Phe Leu Phe Trp Thr Pro Tyr
               235                 240                 245
   aac gtt atg att ttc ctg gag acg ctt aag ctc tat gac ttc ttt ccc      881
   Asn Val Met Ile Phe Leu Glu Thr Leu Lys Leu Tyr Asp Phe Phe Pro
           250                 255                 260
   agt tgt gac atg agg aag gat ctg agg ctg gcc ctc agt gtg act gag      929
   Ser Cys Asp Met Arg Lys Asp Leu Arg Leu Ala Leu Ser Val Thr Glu
       265                 270                 275
   acg gtt gca ttt agc cat tgt tgc ctg aat cct ctc atc tat gca ttt      977
   Thr Val Ala Phe Ser His Cys Cys Leu Asn Pro Leu Ile Tyr Ala Phe
   280                 285                 290                 295
   gct ggg gag aag ttc aga aga tac ctt tac cac ctg tat ggg aaa tgc     1025
   Ala Gly Glu Lys Phe Arg Arg Tyr Leu Tyr His Leu Tyr Gly Lys Cys
                   300                 305                 310
   ctg gct gtc ctg tgt ggg cgc tca gtc cac gtt gat ttc tcc tca tct     1073
   Leu Ala Val Leu Cys Gly Arg Ser Val His Val Asp Phe Ser Ser Ser
               315                 320                 325
   gaa tca caa agg agc agg cat gga agt gtt ctg agc agc aat ttt act     1121
   Glu Ser Gln Arg Ser Arg His Gly Ser Val Leu Ser Ser Asn Phe Thr
           330                 335                 340
   tac cac acg agt gat gga gat gca ttg ctc ctt ctc tga agggaatccc      1170
   Tyr His Thr Ser Asp Gly Asp Ala Leu Leu Leu Leu
       345                 350                 355
   aaagccttgt gtctacagag aacctggagt tcctgaacct gatgctgact agtgaggaaa   1230
   gatttttgtt gttatttctt acaggcacaa                                    1260
<210> 22
   <211> 355
   <212> PRT
   <213> Homo sapiens
<400> 22
   Met Asp Gln Phe Pro Glu Ser Val Thr Glu Asn Phe Glu Tyr Asp Asp
   1               5                   10                  15
   Leu Ala Glu Ala Cys Tyr Ile Gly Asp Ile Val Val Phe Gly Thr Val
               20                  25                  30
   Phe Leu Ser Ile Phe Tyr Ser Val Ile Phe Ala Ile Gly Leu Val Gly
           35                  40                  45
   Asn Leu Leu Val Val Phe Ala Leu Thr Asn Ser Lys Lys Pro Lys Ser
       50                  55                  60
   Val Thr Asp Ile Tyr Leu Leu Asn Leu Ala Leu Ser Asp Leu Leu Phe
   65                  70                  75                  80
   Val Ala Thr Leu Pro Phe Trp Thr His Tyr Leu Ile Asn Glu Lys Gly
                   85                  90                  95
   Leu His Asn Ala Met Cys Lys Phe Thr Thr Ala Phe Phe Phe Ile Gly
   100 105 110
   Phe Phe Gly Ser Ile Phe Phe Ile Thr Val Ile Ser Ile Asp Arg Tyr
   115 120 125
   Leu Ala Ile Val Leu Ala Ala Asn Ser Met Asn Asn Arg Thr Val Gln
   130 135 140
   His Gly Val Thr Ile Ser Leu Gly Val Trp Ala Ala Ala Ile Leu Val
   145 150 155 160
   Ala Ala Pro Gln Phe Met Phe Thr Lys Gln Lys Glu Asn Glu Cys Leu
   165 170 175
   Gly Asp Tyr Pro Glu Val Leu Gln Glu Ile Trp Pro Val Leu Arg Asn
   180 185 190
   Val Glu Thr Asn Phe Leu Gly Phe Leu Leu Pro Leu Leu Ile Met Ser
   195 200 205
   Tyr Cys Tyr Phe Arg Ile Ile Gln Thr Leu Phe Ser Cys Lys Asn His
   210 215 220
   Lys Lys Ala Lys Ala Ile Lys Leu Ile Leu Leu Val Val Ile Val Phe
   225 230 235 240
   Phe Leu Phe Trp Thr Pro Tyr Asn Val Met Ile Phe Leu Glu Thr Leu
   245 250 255
   Lys Leu Tyr Asp Phe Phe Pro Ser Cys Asp Met Arg Lys Asp Leu Arg
   260 265 270
   Leu Ala Leu Ser Val Thr Glu Thr Val Ala Phe Ser His Cys Cys Leu
   275 280 285
   Asn Pro Leu Ile Tyr Ala Phe Ala Gly Glu Lys Phe Arg Arg Tyr Leu
   290 295 300
   Tyr His Leu Tyr Gly Lys Cys Leu Ala Val Leu Cys Gly Arg Ser Val
   305 310 315 320
   His Val Asp Phe Ser Ser Ser Glu Ser Gln Arg Ser Arg His Gly Ser
   325 330 335
   Val Leu Ser Ser Asn Phe Thr Tyr His Thr Ser Asp Gly Asp Ala Leu
   340 345 350
   Leu Leu Leu
   355
<210> 23
   <211> 1560
   <212> DNA
   <213> Homo sapiens
<220>
   <221> CDS
   <222> (387)..(1454)
<400> 23
   aacagattta cttaaccact ggcaaaccac atttcctttt ccagggcaat aacttaaaat     60
   attatgtatt tccctttgtt gcaaagagag gaaatacttc ttcctagact cagggcagct    120
   gtgacccgtc ctcccagaga aatcattaaa ccacaaggat tcagacagag cccagagccc    180
   tgaaaacttt ggccacgcac tttcccgcag cagccacagg caccggcaac ttcagagagc    240
   cagataaaag tggaatgagg aatgcagccg ttctgaacac caccctccat ttcattctgg    300
   aaccgggaag gtacacccag gcatgacaat agcttctctc ctcacagaaa tttaactgat    360
   ttcttcattc tccatttagc aaggtc atg gaa gat ttg gag gaa aca tta ttt     413
                                Met Glu Asp Leu Glu Glu Thr Leu Phe
                                1               5
   gaa gaa ttt gaa aac tat tcc tat gac cta gac tat tac tct ctg gag      461
   Glu Glu Phe Glu Asn Tyr Ser Tyr Asp Leu Asp Tyr Tyr Ser Leu Glu
   10                  15                  20                  25
   tct gat ttg gag gag aaa gtc cag ctg gga gtt gtt cac tgg gtc tcc      509
   Ser Asp Leu Glu Glu Lys Val Gln Leu Gly Val Val His Trp Val Ser
                   30                  35                  40
   ctg gtg tta tat tgt ttg gct ttt gtt ctg gga att cca gga aat gcc      557
   Leu Val Leu Tyr Cys Leu Ala Phe Val Leu Gly Ile Pro Gly Asn Ala
               45                  50                  55
   atc gtc att tgg ttc acg ggg ttc aag tgg aag aag aca gtc acc act      605
   Ile Val Ile Trp Phe Thr Gly Phe Lys Trp Lys Lys Thr Val Thr Thr
           60                  65                  70
   ctg tgg ttc ctc aat cta gcc att gcg gat ttc att ttt ctt ctc ttt      653
   Leu Trp Phe Leu Asn Leu Ala Ile Ala Asp Phe Ile Phe Leu Leu Phe
       75                  80                  85
   ctg ccc ctg tac atc tcc tat gtg gcc atg aat ttc cac tgg ccc ttt      701
   Leu Pro Leu Tyr Ile Ser Tyr Val Ala Met Asn Phe His Trp Pro Phe
   90                  95                  100                 105
   ggc atc tgg ctg tgc aaa gcc aat tcc ttc act gcc cag ttg aac atg      749
   Gly Ile Trp Leu Cys Lys Ala Asn Ser Phe Thr Ala Gln Leu Asn Met
                   110                 115                 120
   ttt gcc agt gtt ttt ttc ctg aca gtg atc agc ctg gac cac tat atc      797
   Phe Ala Ser Val Phe Phe Leu Thr Val Ile Ser Leu Asp His Tyr Ile
               125                 130                 135
   cac ttg atc cat cct gtc tta tct cat cgg cat cga acc ctc aag aac      845
   His Leu Ile His Pro Val Leu Ser His Arg His Arg Thr Leu Lys Asn
           140                 145                 150
   tct ctg att gtc att ata ttc atc tgg ctt ttg gct tct cta att ggc       893
   Ser Leu Ile Val Ile Ile Phe Ile Trp Leu Leu Ala Ser Leu Ile Gly
       155                 160                 165
   ggt cct gcc ctg tac ttc cgg gac act gtg gag ttc aat aat cat act       941
   Gly Pro Ala Leu Tyr Phe Arg Asp Thr Val Glu Phe Asn Asn His Thr
   170                 175                 180                 185
   ctt tgc tat aac aat ttt cag aag cat gat cct gac ctc act ttg atc       989
   Leu Cys Tyr Asn Asn Phe Gln Lys His Asp Pro Asp Leu Thr Leu Ile
                   190                 195                 200
   agg cac cat gtt ctg act tgg gtg aaa ttt atc att ggc tat ctc ttc      1037
   Arg His His Val Leu Thr Trp Val Lys Phe Ile Ile Gly Tyr Leu Phe
               205                 210                 215
   cct ttg cta aca atg agt att tgc tac ttg tgt ctc atc ttc aag gtg      1085
   Pro Leu Leu Thr Met Ser Ile Cys Tyr Leu Cys Leu Ile Phe Lys Val
           220                 225                 230
   aag aag cga agc atc ctg atc tcc agt agg cat ttc tgg aca att ctg      1133
   Lys Lys Arg Ser Ile Leu Ile Ser Ser Arg His Phe Trp Thr Ile Leu
       235                 240                 245
   gtt gtg gtt gtg gcc ttt gtg gtt tgc tgg act cct tat cac ctg ttt      1181
   Val Val Val Val Ala Phe Val Val Cys Trp Thr Pro Tyr His Leu Phe
   250                 255                 260                 265
   agc att tgg gag ctc acc att cac cac aat agc tat tcc cac cat gtg      1229
   Ser Ile Trp Glu Leu Thr Ile His His Asn Ser Tyr Ser His His Val
                   270                 275                 280
   atg cag gct gga atc ccc ctc tcc act ggt ttg gca ttc ctc aat agt      1277
   Met Gln Ala Gly Ile Pro Leu Ser Thr Gly Leu Ala Phe Leu Asn Ser
               285                 290                 295
   tgc ttg aac ccc atc ctt tat gtc cta att agt aag aag ttc caa gct      1325
   Cys Leu Asn Pro Ile Leu Tyr Val Leu Ile Ser Lys Lys Phe Gln Ala
           300                 305                 310
   cgc ttc cgg tcc tca gtt gct gag ata ctc aag tac aca ctg tgg gaa      1373
   Arg Phe Arg Ser Ser Val Ala Glu Ile Leu Lys Tyr Thr Leu Trp Glu
       315                 320                 325
   gtc agc tgt tct ggc aca gtg agt gaa cag ctc agg aac tca gaa acc      1421
   Val Ser Cys Ser Gly Thr Val Ser Glu Gln Leu Arg Asn Ser Glu Thr
   330                 335                 340                 345
   aag aat ctg tgt ctc ctg gaa aca gct caa taa gttattactt ttccacaaat    1474
   Lys Asn Leu Cys Leu Leu Glu Thr Ala Gln
                   350                 355
   cagtatatgg ctttttatgt gggtcctctg actgatgctt tcagattaaa attgtttcca    1534
   agatagagag ccgactccac tttcat                                         1560
<210> 24
   <211> 355
   <212> PRT
   <213> Homo sapiens
<400> 24
   Met Glu Asp Leu Glu Glu Thr Leu Phe Glu Glu Phe Glu Asn Tyr Ser
   1               5                   10                  15
   Tyr Asp Leu Asp Tyr Tyr Ser Leu Glu Ser Asp Leu Glu Glu Lys Val
               20                  25                  30
   Gln Leu Gly Val Val His Trp Val Ser Leu Val Leu Tyr Cys Leu Ala
           35                  40                  45
   Phe Val Leu Gly Ile Pro Gly Asn Ala Ile Val Ile Trp Phe Thr Gly
       50                  55                  60
   Phe Lys Trp Lys Lys Thr Val Thr Thr Leu Trp Phe Leu Asn Leu Ala
   65                  70                  75                  80
   Ile Ala Asp Phe Ile Phe Leu Leu Phe Leu Pro Leu Tyr Ile Ser Tyr
                   85                  90                  95
   Val Ala Met Asn Phe His Trp Pro Phe Gly Ile Trp Leu Cys Lys Ala
               100                 105                 110
   Asn Ser Phe Thr Ala Gln Leu Asn Met Phe Ala Ser Val Phe Phe Leu
           115                 120                 125
   Thr Val Ile Ser Leu Asp His Tyr Ile His Leu Ile His Pro Val Leu
       130                 135                 140
   Ser His Arg His Arg Thr Leu Lys Asn Ser Leu Ile Val Ile Ile Phe
   145                 150                 155                 160
   Ile Trp Leu Leu Ala Ser Leu Ile Gly Gly Pro Ala Leu Tyr Phe Arg
                   165                 170                 175
   Asp Thr Val Glu Phe Asn Asn His Thr Leu Cys Tyr Asn Asn Phe Gln
               180                 185                 190
   Lys His Asp Pro Asp Leu Thr Leu Ile Arg His His Val Leu Thr Trp
           195                 200                 205
   Val Lys Phe Ile Ile Gly Tyr Leu Phe Pro Leu Leu Thr Met Ser Ile
       210                 215                 220
   Cys Tyr Leu Cys Leu Ile Phe Lys Val Lys Lys Arg Ser Ile Leu Ile
   225                 230                 235                 240
   Ser Ser Arg His Phe Trp Thr Ile Leu Val Val Val Val Ala Phe Val
                   245                 250                 255
   Val Cys Trp Thr Pro Tyr His Leu Phe Ser Ile Trp Glu Leu Thr Ile
               260                 265                 270
   His His Asn Ser Tyr Ser His His Val Met Gln Ala Gly Ile Pro Leu
           275                 280                 285
   Ser Thr Gly Leu Ala Phe Leu Asn Ser Cys Leu Asn Pro Ile Leu Tyr
       290                 295                 300
   Val Leu Ile Ser Lys Lys Phe Gln Ala Arg Phe Arg Ser Ser Val Ala
   305                 310                 315                 320
   Glu Ile Leu Lys Tyr Thr Leu Trp Glu Val Ser Cys Ser Gly Thr Val
                   325                 330                 335
   Ser Glu Gln Leu Arg Asn Ser Glu Thr Lys Asn Leu Cys Leu Leu Glu
               340                 345                 350
   Thr Ala Gln
           355
<210> 25
   <211> 1083
   <212> DNA
   <213> Homo sapiens
<220>
   <221> CDS
   <222> (1)..(1083)
<400> 25
   atg gac cca gaa gaa act tca gtt tat ttg gat tat tac tat gct acg       48
   Met Asp Pro Glu Glu Thr Ser Val Tyr Leu Asp Tyr Tyr Tyr Ala Thr
   1               5                   10                  15
   agc cca aac tct gac atc agg gag acc cac tcc cat gtt cct tac acc       96
   Ser Pro Asn Ser Asp Ile Arg Glu Thr His Ser His Val Pro Tyr Thr
               20                  25                  30
   tct gtc ttc ctt cca gtc ttt tac aca gct gtg ttc ctg act gga gtg      144
   Ser Val Phe Leu Pro Val Phe Tyr Thr Ala Val Phe Leu Thr Gly Val
           35                  40                  45
   ctg ggg aac ctt gtt ctc atg gga gcg ttg cat ttc aaa ccc ggc agc      192
   Leu Gly Asn Leu Val Leu Met Gly Ala Leu His Phe Lys Pro Gly Ser
       50                  55                  60
   cga aga ctg atc gac atc ttt atc atc aat ctg gct gcc tct gac ttc      240
   Arg Arg Leu Ile Asp Ile Phe Ile Ile Asn Leu Ala Ala Ser Asp Phe
   65                  70                  75                  80
   att ttt ctt gtc aca ttg cct ctc tgg gtg gat aaa gaa gca tct cta      288
   Ile Phe Leu Val Thr Leu Pro Leu Trp Val Asp Lys Glu Ala Ser Leu
                   85                  90                  95
   gga ctg tgg agg acg ggc tcc ttc ctg tgc aaa ggg agc tcc tac atg      336
   Gly Leu Trp Arg Thr Gly Ser Phe Leu Cys Lys Gly Ser Ser Tyr Met
               100                 105                 110
   atc tcc gtc aat atg cac tgc agt gtc ctc ctg ctc act tgc atg agt      384
   Ile Ser Val Asn Met His Cys Ser Val Leu Leu Leu Thr Cys Met Ser
           115                 120                 125
   gtt gac cgc tac ctg gcc att gtg tgg cca gtc gta tcc agg aaa ttc      432
   Val Asp Arg Tyr Leu Ala Ile Val Trp Pro Val Val Ser Arg Lys Phe
       130                 135                 140
   aga agg aca gac tgt gca tat gta gtc tgt gcc agc atc tgg ttt atc      480
   Arg Arg Thr Asp Cys Ala Tyr Val Val Cys Ala Ser Ile Trp Phe Ile
   145                 150                 155                 160
   tcc tgc ctg ctg ggg ttg cct act ctt ctg tcc agg gag ctc acg ctg      528
   Ser Cys Leu Leu Gly Leu Pro Thr Leu Leu Ser Arg Glu Leu Thr Leu
               165                 170                 175
   att gat gat aag cca tac tgt gca gag aaa aag gca act cca att aaa      576
   Ile Asp Asp Lys Pro Tyr Cys Ala Glu Lys Lys Ala Thr Pro Ile Lys
               180                 185                 190
   ctc ata tgg tcc ctg gtg gcc tta att ttc acc ttt ttt gtc cct ttg      624
   Leu Ile Trp Ser Leu Val Ala Leu Ile Phe Thr Phe Phe Val Pro Leu
           195                 200                 205
   ttg agc att gtg acc tgc tac tgt tgc att gca agg aag ctg tgt gcc      672
   Leu Ser Ile Val Thr Cys Tyr Cys Cys Ile Ala Arg Lys Leu Cys Ala
       210                 215                 220
   cat tac cag caa tca gga aag cac aac aaa aag ctg aag aaa tct ata      720
   His Tyr Gln Gln Ser Gly Lys His Asn Lys Lys Leu Lys Lys Ser Ile
   225                 230                 235                 240
   aag atc atc ttt att gtc gtg gca gcc ttt ctt gtc tcc tgg ctg ccc      768
   Lys Ile Ile Phe Ile Val Val Ala Ala Phe Leu Val Ser Trp Leu Pro
                   245                 250                 255
   ttc aat act ttc aag ttc ctg gcc att gtc tct ggg ttg cgg caa gaa      816
   Phe Asn Thr Phe Lys Phe Leu Ala Ile Val Ser Gly Leu Arg Gln Glu
               260                 265                 270
   cac tat tta ccc tca gct att ctt cag ctt ggt atg gag gtg agt gga      864
   His Tyr Leu Pro Ser Ala Ile Leu Gln Leu Gly Met Glu Val Ser Gly
           275                 280                 285
   ccc ttg gca ttt gcc aac agc tgt gtc aac cct ttc att tac tat atc      912
   Pro Leu Ala Phe Ala Asn Ser Cys Val Asn Pro Phe Ile Tyr Tyr Ile
       290                 295                 300
   ttc gac agc tac atc cgc cgg gcc att gtc cac tgc ttg tgc cct tgc      960
   Phe Asp Ser Tyr Ile Arg Arg Ala Ile Val His Cys Leu Cys Pro Cys
   305                 310                 315                 320
   ctg aaa aac tat gac ttt ggg agt agc act gag aca tca gat agt cac     1008
   Leu Lys Asn Tyr Asp Phe Gly Ser Ser Thr Glu Thr Ser Asp Ser His
                   325                 330                 335
   ctc act aag gct ctc tcc acc ttc att cat gca gaa gat ttt gcc agg     1056
   Leu Thr Lys Ala Leu Ser Thr Phe Ile His Ala Glu Asp Phe Ala Arg
               340                 345                 350
   agg agg aag agg tct gtg tca ctc taa                                 1083
   Arg Arg Lys Arg Ser Val Ser Leu
   355 360
<210> 26
   <211> 360
   <212> PRT
   <213> Homo sapiens
<400> 26
   Met Asp Pro Glu Glu Thr Ser Val Tyr Leu Asp Tyr Tyr Tyr Ala Thr
   1               5                   10                  15
   Ser Pro Asn Ser Asp Ile Arg Glu Thr His Ser His Val Pro Tyr Thr
               20                  25                  30
   Ser Val Phe Leu Pro Val Phe Tyr Thr Ala Val Phe Leu Thr Gly Val
           35                  40                  45
   Leu Gly Asn Leu Val Leu Met Gly Ala Leu His Phe Lys Pro Gly Ser
       50                  55                  60
   Arg Arg Leu Ile Asp Ile Phe Ile Ile Asn Leu Ala Ala Ser Asp Phe
   65                  70                  75                  80
   Ile Phe Leu Val Thr Leu Pro Leu Trp Val Asp Lys Glu Ala Ser Leu
                   85                  90                  95
   Gly Leu Trp Arg Thr Gly Ser Phe Leu Cys Lys Gly Ser Ser Tyr Met
               100                 105                 110
   Ile Ser Val Asn Met His Cys Ser Val Leu Leu Leu Thr Cys Met Ser
           115                 120                 125
   Val Asp Arg Tyr Leu Ala Ile Val Trp Pro Val Val Ser Arg Lys Phe
       130                 135                 140
   Arg Arg Thr Asp Cys Ala Tyr Val Val Cys Ala Ser Ile Trp Phe Ile
   145                 150                 155                 160
   Ser Cys Leu Leu Gly Leu Pro Thr Leu Leu Ser Arg Glu Leu Thr Leu
                   165                 170                 175
   Ile Asp Asp Lys Pro Tyr Cys Ala Glu Lys Lys Ala Thr Pro Ile Lys
               180                 185                 190
   Leu Ile Trp Ser Leu Val Ala Leu Ile Phe Thr Phe Phe Val Pro Leu
           195                 200                 205
   Leu Ser Ile Val Thr Cys Tyr Cys Cys Ile Ala Arg Lys Leu Cys Ala
       210                 215                 220
   His Tyr Gln Gln Ser Gly Lys His Asn Lys Lys Leu Lys Lys Ser Ile
   225 230 235 240
   Lys Ile Ile Phe Ile Val Val Ala Ala Phe Leu Val Ser Trp Leu Pro
   245 250 255
   Phe Asn Thr Phe Lys Phe Leu Ala Ile Val Ser Gly Leu Arg Gln Glu
   260 265 270
   His Tyr Leu Pro Ser Ala Ile Leu Gln Leu Gly Met Glu Val Ser Gly
   275 280 285
   Pro Leu Ala Phe Ala Asn Ser Cys Val Asn Pro Phe Ile Tyr Tyr Ile
   290 295 300
   Phe Asp Ser Tyr Ile Arg Arg Ala Ile Val His Cys Leu Cys Pro Cys
   305 310 315 320
   Leu Lys Asn Tyr Asp Phe Gly Ser Ser Thr Glu Thr Ser Asp Ser His
   325 330 335
   Leu Thr Lys Ala Leu Ser Thr Phe Ile His Ala Glu Asp Phe Ala Arg
   340 345 350
   Arg Arg Lys Arg Ser Val Ser Leu
   355 360
<210> 27
   <211> 1680
   <212> DNA
   <213> Homo sapiens
<220>
   <221> CDS
   <222> (450)..(1592)
<400> 27
   ggaaagccga cttgcaaaac cacagataat gttcagccca gcacagtagg ggtcaatttg     60
   gtccacttgc tcagtgacaa aaagaaaaaa aaagtgggct gtcactaaag attttgactc    120
   acaagagagg ggctggtctg gaggtgggag gagggagtga cgagtcaagg aggagacagg    180
   gacgcaggag ggtgcaagga agtgtcttaa ctgagacggg ggtaaggcaa gagagggtgg    240
   aggaaattct gcaggagaca ggcttcctcc agggtctgga gaacccagag gcagctcctc    300
   ctgagtgctg ggaaggactc tgggcatctt cagcccttct tactctctga ggctcaagcc    360
   agaaattcag gctgcttgca gagtgggtga cagagccacg gagctggtgt ccctgggacc    420
   ctctgcccgt cttctctcca ctccccagc atg gag gaa ggt ggt gat ttt gac      473
                                   Met Glu Glu Gly Gly Asp Phe Asp
                                   1               5
   aac tac tat ggg gca gac aac cag tct gag tgt gag tac aca gac tgg      521
   Asn Tyr Tyr Gly Ala Asp Asn Gln Ser Glu Cys Glu Tyr Thr Asp Trp
       10                  15                  20
   aaa tcc tcg ggg gcc ctc atc cct gcc atc tac atg ttg gtc ttc ctc      569
   Lys Ser Ser Gly Ala Leu Ile Pro Ala Ile Tyr Met Leu Val Phe Leu
   25                  30                  35                  40
   ctg ggc acc acg ggc aac ggt ctg gtg ctc tgg acc gtg ttt cgg agc      617
   Leu Gly Thr Thr Gly Asn Gly Leu Val Leu Trp Thr Val Phe Arg Ser
                   45                  50                  55
   agc cgg gag aag agg cgc tca gct gat atc ttc att gct agc ctg gcg      665
   Ser Arg Glu Lys Arg Arg Ser Ala Asp Ile Phe Ile Ala Ser Leu Ala
               60                  65                  70
   gtg gct gac ctg acc ttc gtg gtg acg ctg ccc ctg tgg gct acc tac      713
   Val Ala Asp Leu Thr Phe Val Val Thr Leu Pro Leu Trp Ala Thr Tyr
           75                  80                  85
   acg tac cgg gac tat gac tgg ccc ttt ggg acc ttc ttc tgc aag ctc      761
   Thr Tyr Arg Asp Tyr Asp Trp Pro Phe Gly Thr Phe Phe Cys Lys Leu
       90                  95                  100
   agc agc tac ctc atc ttc gtc aac atg tac gcc agc gtc ttc tgc ctc      809
   Ser Ser Tyr Leu Ile Phe Val Asn Met Tyr Ala Ser Val Phe Cys Leu
   105                 110                 115                 120
   acc ggc ctc agc ttc gac cgc tac ctg gcc atc gtg agg cca gtg gcc      857
   Thr Gly Leu Ser Phe Asp Arg Tyr Leu Ala Ile Val Arg Pro Val Ala
                   125                 130                 135
   aat gct cgg ctg agg ctg cgg gtc agc ggg gcc gtg gcc acg gca gtt      905
   Asn Ala Arg Leu Arg Leu Arg Val Ser Gly Ala Val Ala Thr Ala Val
               140                 145                 150
   ctt tgg gtg ctg gcc gcc ctc ctg gcc atg cct gtc atg gtg tta cgc      953
   Leu Trp Val Leu Ala Ala Leu Leu Ala Met Pro Val Met Val Leu Arg
           155                 160                 165
   acc acc ggg gac ttg gag aac acc act aag gtg cag tgc tac atg gac     1001
   Thr Thr Gly Asp Leu Glu Asn Thr Thr Lys Val Gln Cys Tyr Met Asp
       170                 175                 180
   tac tcc atg gtg gcc act gtg agc tca gag tgg gcc tgg gag gtg ggc     1049
   Tyr Ser Met Val Ala Thr Val Ser Ser Glu Trp Ala Trp Glu Val Gly
   185                 190                 195                 200
   ctt ggg gtc tcg tcc acc acc gtg ggc ttt gtg gtg ccc ttc acc atc     1097
   Leu Gly Val Ser Ser Thr Thr Val Gly Phe Val Val Pro Phe Thr Ile
                   205                 210                 215
   atg ctg acc tgt tac ttc ttc atc gcc caa acc atc gct ggc cac ttc     1145
   Met Leu Thr Cys Tyr Phe Phe Ile Ala Gln Thr Ile Ala Gly His Phe
               220                 225                 230
   cgc aag gaa cgc atc gag ggc ctg cgg aag cgg cgc cgg ctg ctc agc     1193
   Arg Lys Glu Arg Ile Glu Gly Leu Arg Lys Arg Arg Arg Leu Leu Ser
           235                 240                 245
   atc atc gtg gtg ctg gtg gtg acc ttt gcc ctg tgc tgg atg ccc tac     1241
   Ile Ile Val Val Leu Val Val Thr Phe Ala Leu Cys Trp Met Pro Tyr
       250                 255                 260
   cac ctg gtg aag acg ctg tac atg ctg ggc agc ctg ctg cac tgg ccc      1289
   His Leu Val Lys Thr Leu Tyr Met Leu Gly Ser Leu Leu His Trp Pro
   265                 270                 275                 280
   tgt gac ttt gac ctc ttc ctc atg aac atc ttc ccc tac tgc acc tgc      1337
   Cys Asp Phe Asp Leu Phe Leu Met Asn Ile Phe Pro Tyr Cys Thr Cys
                   285                 290                 295
   atc agc tac gtc aac agc tgc ctc aac ccc ttc ctc tat gcc ttt ttc      1385
   Ile Ser Tyr Val Asn Ser Cys Leu Asn Pro Phe Leu Tyr Ala Phe Phe
               300                 305                 310
   gac ccc cgc ttc cgc cag gcc tgc acc tcc atg ctc tgc tgt ggc cag      1433
   Asp Pro Arg Phe Arg Gln Ala Cys Thr Ser Met Leu Cys Cys Gly Gln
           315                 320                 325
   agc agg tgc gca ggc acc tcc cac agc agc agt ggg gag aag tca gcc      1481
   Ser Arg Cys Ala Gly Thr Ser His Ser Ser Ser Gly Glu Lys Ser Ala
       330                 335                 340
   agc tac tct tcg ggg cac agc cag ggg ccc ggc ccc aac atg ggc aag      1529
   Ser Tyr Ser Ser Gly His Ser Gln Gly Pro Gly Pro Asn Met Gly Lys
   345                 350                 355                 360
   ggt gga gaa cag atg cac gag aaa tcc atc ccc tac agc cag gag acc      1577
   Gly Gly Glu Gln Met His Glu Lys Ser Ile Pro Tyr Ser Gln Glu Thr
                   365                 370                 375
   ctt gtg gtt gac tag ggctgggagc agagagaagc ctggcgccct cggccctccc      1632
   Leu Val Val Asp
               380
   cggcctttgc ccttgctttc tgaaaatcag gtagtgtggc tactcctt                 1680
<210> 28
   <211> 380
   <212> PRT
   <213> Homo sapiens
<400> 28
   Met Glu Glu Gly Gly Asp Phe Asp Asn Tyr Tyr Gly Ala Asp Asn Gln
   1 5 10 15
   Ser Glu Cys Glu Tyr Thr Asp Trp Lys Ser Ser Gly Ala Leu Ile Pro
   20 25 30
   Ala Ile Tyr Met Leu Val Phe Leu Leu Gly Thr Thr Gly Asn Gly Leu
   35 40 45
   Val Leu Trp Thr Val Phe Arg Ser Ser Arg Glu Lys Arg Arg Ser Ala
   50 55 60
   Asp Ile Phe Ile Ala Ser Leu Ala Val Ala Asp Leu Thr Phe Val Val
   65 70 75 80
   Thr Leu Pro Leu Trp Ala Thr Tyr Thr Tyr Arg Asp Tyr Asp Trp Pro
   85 90 95
   Phe Gly Thr Phe Phe Cys Lys Leu Ser Ser Tyr Leu Ile Phe Val Asn
   100 105 110
   Met Tyr Ala Ser Val Phe Cys Leu Thr Gly Leu Ser Phe Asp Arg Tyr
   115 120 125
   Leu Ala Ile Val Arg Pro Val Ala Asn Ala Arg Leu Arg Leu Arg Val
   130 135 140
   Ser Gly Ala Val Ala Thr Ala Val Leu Trp Val Leu Ala Ala Leu Leu
   145 150 155 160
   Ala Met Pro Val Met Val Leu Arg Thr Thr Gly Asp Leu Glu Asn Thr
   165 170 175
   Thr Lys Val Gln Cys Tyr Met Asp Tyr Ser Met Val Ala Thr Val Ser
   180 185 190
   Ser Glu Trp Ala Trp Glu Val Gly Leu Gly Val Ser Ser Thr Thr Val
   195 200 205
   Gly Phe Val Val Pro Phe Thr Ile Met Leu Thr Cys Tyr Phe Phe Ile
   210 215 220
   Ala Gln Thr Ile Ala Gly His Phe Arg Lys Glu Arg Ile Glu Gly Leu
   225 230 235 240
   Arg Lys Arg Arg Arg Leu Leu Ser Ile Ile Val Val Leu Val Val Thr
   245 250 255
   Phe Ala Leu Cys Trp Met Pro Tyr His Leu Val Lys Thr Leu Tyr Met
   260 265 270
   Leu Gly Ser Leu Leu His Trp Pro Cys Asp Phe Asp Leu Phe Leu Met
   275 280 285
   Asn Ile Phe Pro Tyr Cys Thr Cys Ile Ser Tyr Val Asn Ser Cys Leu
   290 295 300
   Asn Pro Phe Leu Tyr Ala Phe Phe Asp Pro Arg Phe Arg Gln Ala Cys
   305 310 315 320
   Thr Ser Met Leu Cys Cys Gly Gln Ser Arg Cys Ala Gly Thr Ser His
   325 330 335
   Ser Ser Ser Gly Glu Lys Ser Ala Ser Tyr Ser Ser Gly His Ser Gln
   340 345 350
   Gly Pro Gly Pro Asn Met Gly Lys Gly Gly Glu Gln Met His Glu Lys
           355                     360                     365
   Ser Ile Pro Tyr Ser Gln Glu Thr Leu Val Val Asp
       370                     375                 380
<210> 29
   <211> 1560
   <212> DNA
   <213> Homo sapiens
<220>
   <221> CDS
   <222> (355)..(1476)
<400> 29
   gaattcggca cgagccccgg cggccagcag ggagctcagg acagagcagg ctccctggga     60
   agcctccggg tgataggggt gttccagctg cggcgctctg ggggttcaga gggggatctt    120
   gaatgaacaa atgaatgaac tgctttctgg gcaaacagcc acagccagag gagcctgtga    180
   ttggcagaaa gaagccaggg tgtgcaagtc tccccaacag cctcgagtgg cctgcagtca    240
   cagggaaccc tcaggaagac cttccgggca gagaccagag ggtgtttcta gctgtgtaca    300
   gggactgatt ggctgaggac tcacattgga gagctgcaga caacataacg gtga atg     357
   aga atg gag gat gaa gat tac aac act tcc atc agt tac ggt gat gaa     405
   tac cct gat tat tta gac tcc att gtg gtt ttg gag gac tta tcc ccc     453
   ttg gaa gcc agg gtg acc agg atc ttc ctg gtg gtg gtc tac agc atc     501
   gtc tgc ttc ctc ggg att ctg ggc aat ggt ctg gtg atc atc att gcc     549
   acc ttc aag atg aag aag aca gtg aac atg gtc tgg ttc ctc aac ctg     597
   gca gtg gca gat ttc ctg ttc aac gtc ttc ctc cca atc cat atc acc     645
   tat gcc gcc atg gac tac cac tgg gtt ttc ggg aca gcc atg tgc aag     693
   atc agc aac ttc ctt ctc atc cac aac atg ttc acc agc gtc ttc ctg     741
   ctg acc atc atc agc tct gac cgc tgc atc tct gtg ctc ctc cct gtc      789
   tgg tcc cag aac cac cgc agc gtt cgc ctg gct tac atg gcc tgc atg      837
   gtc atc tgg gtc ctg gct ttc ttc ttg agt tcc cca tct ctc gtc ttc      885
   cgg gac aca gcc aac ctg cat ggg aaa ata tcc tgc ttc aac aac ttc      933
   agc ctg tcc aca cct ggg tct tcc tcg tgg ccc act cac tcc caa atg      981
   gac cct gtg ggg tat agc cgg cac atg gtg gtg act gtc acc cgc ttc      1029
   ctc tgt ggc ttc ctg gtc cca gtc ctc atc atc aca gct tgc tac ctc      1077
   acc atc gtc tgc aaa ctg cac cgc aac cgc ctg gcc aag acc aag aag      1125
   ccc ttc aag att att gtg acc atc atc att acc ttc ttc ctc tgc tgg      1173
   tgc ccc tac cac aca ctc aac ctc cta gag ctc cac cac act gcc atg      1221
   cct ggc tct gtc ttc agc ctg ggt ttg ccc ctg gcc act gcc ctt gcc      1269
   att gcc aac agc tgc atg aac ccc att ctg tat gtt ttc atg ggt cag      1317
   gac ttc aag aag ttc aag gtg gcc ctc ttc tct cgc ctg gtc aat gct      1365
   cta agt gaa gat aca ggc cac tct tcc tac ccc agc cat aga agc ttt      1413
   acc aag atg tca tca atg aat gag agg act tct atg aat gag agg gag      1461
   acc ggc atg ctt tga tcctcactgt ggaacccctc aatggactct ctcaacccag      1516
   ggacacccaa ggatatgtct tctgaagatc aaggcaagaa cctc                     1560
<210> 30
   <211> 373
   <212> PRT
   <213> Homo sapiens
<400> 30
<210> 31
   <211> 23
   <212> RNA
   <213> Homo sapiens
<400> 31
   ugagugugug ugugugagug ugu 23
<210> 32
   <211> 22
   <212> RNA
   <213> Homo sapiens
<400> 32
   aggcggggcg ccgcgggacc gc 22
<210> 33
   <211> 21
   <212> RNA
   <213> Homo sapiens
<400> 33
   agggagggac gggggcugug c 21
<210> 34
   <211> 19
   <212> RNA
   <213> Homo sapiens
<400> 34
   gagccaguug gacaggagc 19
<210> 35
   <211> 25
   <212> RNA
   <213> Homo sapiens
<400> 35
   agggaucgcg ggcggguggc ggccu 25
<210> 36
   <211> 23
   <212> RNA
   <213> Homo sapiens
<400> 36
   aacauucauu.gcugucggug ggu 23
<210> 37
   <211> 22
   <212> RNA
   <213> Homo sapiens
<400> 37
   ugagguagua guuuguacag uu 22
<210> 38
   <211> 22
   <212> RNA
   <213> Homo sapiens
<400> 38
   uguaaacauc cucgacugga ag 22
<210> 39
   <211> 22
   <212> RNA
   <213> Homo sapiens
<400> 39
   ucagugcacu acagaacuuu gu 22
<210> 40
   <211> 22
   <212> RNA
   <213> Homo sapiens
<400> 40
   auaaagcuag auaaccgaaa gu 22